# EUROPEAN PATENT SPECIFICATION

(11) **EP 2 943 138 B1**
(45) Date of publication and mention of the grant of the patent: **25.05.2016**
(21) Application number: 15701784.9
(22) Date of filing: 29.01.2015
(51) Int. Cl.: A61B 18/14, A61N 1/40

(54) **SHAPE-ADAPTING ELECTRODE FOR ELECTROMAGNETIC ENERGY TRANSFER**
ELEKTRODE MIT FORMANPASSUNG FÜR ELEKTROMAGNETISCHE ENERGIEÜBERTRAGUNG
ÉLECTRODE D'ADAPTATION DE FORME POUR LE TRANSFERT D'ÉNERGIE ÉLECTROMAGNÉTIQUE

(30) Priority: 30.01.2014 EP 14153349
(43) Date of publication of application: 18.11.2015
(73) Proprietor: Oncotherm Kft., 2071 Páty (HU)
(72) Inventor: ANDOCS, Gabor, 2030 Erd (HU); SZÁSZ, Andras, 2071 Páty (HU); SZÁSZ, Olivér, 2071 Páty (HU)
(74) Representative: Arth, Hans-Lothar
(86) International application number: PCT/EP2015/051778
(87) International publication number: WO 2015/114038

(56) References cited:
- EP-A2- 0 713 691
- WO-A1-98/05286
- US-A- 5 945 827
- US-A1- 2004 044 341

## Description

The present invention is related to a radiofrequency electrode (RF electrode) for the use in hyperthermia that can be fitted to the shape of a target to be treated (e.g. a patient) and that is also able to reversibly retain the shape it has been fitted to. Thereby, a reliable and close contact between the target to be treated (e.g. a patient) and the inventive RF electrode is established that enables an efficient and safe energy transfer to the target to be treated during a hyperthermia treatment of various medical conditions or for cosmetic purposes.

### Background of the invention

Heating is widely applied in many areas of medicine and also used for cosmetic treatments. For example radiofrequency/microwave hyperthermia devices can be used to force energy absorption in tissues to cause damage to unwanted structures and/or increase the temperature of a targeted area above the normal body temperature. One use of hyperthermia devices is the treatment of cancer. In the case of cancer treatment, tumour cells are more sensitive to stresses, for example, heat and/or electromagnetic fields and chemical treatments, than the surrounding healthy cells. Therefore, the aim is to pump energy to the tumour tissue, which is sufficient to irreversibly damage the tumour cells, but which can be tolerated by healthy cells. Another use for hyperthermia devices is to increase temperature and blood circulation for cosmetic (fat-burning, lipid-distortion, shape-correction, etc), dermatological and pain relief treatments.

The use of hyperthermia treatment for the cure of diseases is known since ancient times, but it has gained importance in the latest decades. There are numerous studies showing proof of efficiency of the use of hyperthermia in the treatment of cancer, especially in combination with conventional cancer therapies, such as radiotherapy, chemotherapy and surgery (Review: Van der Zee, J.; Heating the patient: a promising approach?; Annals of Oncology 13; 2002; pages 1173-1184).

One can roughly distinguish three operating modes of hyperthermia treatment:
In **local hyperthermia,** only a very restricted area of the patient's body, in case of cancer the cancerous tissue, is heated by various techniques. These include the use of energy in form of microwaves, radiofrequency or ultrasound. A special variant of local hyperthermia is the so-called radiofrequency ablation (RFA), by which sites (e.g. tumours) deep inside the body can be treated. In RFA, a probe (e.g. a needle) is placed by an invasive procedure under anaesthesia and under visual guidance inside the target tissue and is subsequently heated by application of radio frequency (RF) waves. Thus, the RFA method is an invasive method using radiofrequency waves for burning-out a lesion, thereby causing vehement necrosis.
In **regional hyperthermia,** larger areas of the body (e.g. an organ, a limb) are heated by various means including the methods of local hyperthermia treatment, as mentioned above, and also the perfusion of the peritoneal cavity with warm solutions. Alternatively, a fraction of the blood of a patient can be removed from his circulatory system, heated, and then re-perfused into the targeted limb or organ.
In **whole body hyperthermia** more or less the whole body of a patient is subjected to a heat treatment resulting in an elevated body temperature. There are various conventional means to increase the temperature of the whole body including immersion in a heated bath, irradiative treatment for example in an infrared cabin as well as the use of heated beds or blankets.

A general requirement for hyperthermia treatment is a selectivity of the treatment as high as possible. That means it is desired to selectively heat the target tissue/cell in order to destroy or support the destruction of the target tissue/cell, while the deterioration of surrounding healthy tissue should be reduced to a minimum.

The inventive RF electrode for hyperthermia treatment is favourably incorporated into a capacitor arrangement that uses capacitive coupling between the electrodes and a RF current which also runs through the target tissue of the patient while the body part of the patient between the electrodes acts like a dielectric material wherein the target tissue is heated by Joule heat (Q=I²R) generated by conversion of the current flow through the target tissue into heat as well as by the potential difference used for an electric field effect. Selectivity of the generation of heat mostly within the target tissue or the diseased tissue and not the healthy tissue is achieved by using conductivity differences of the healthy tissue in regard to the diseased or target tissue. The target tissue such as a malignant tumor tissue has a higher complex or overall conductivity (admittance) than healthy tissue and consequently has a higher absorption rate of the current going through it in comparison to healthy or normal tissue so that the Joule heat is mostly generated when the current passes the target tissue. The hyperthermia method used in the device of the present invention is thus also referred to as **"conductive hyperthermia"** in order to point to the important differences to radiative hyperthermia which uses an antenna arrangement and most often ablation and has a high risk of causing necrosis. Using said conductivity differences of the respective tissues leads to an automatic selection of the focus. This has immediate consequences on expansible organs like the lung or the heart, or if the patient moves during a treatment session which may exceed one hour. While the focus in the conventional device remains at the spot on which it was focused before, independent from the actual position of the tumour, the present invention follows any movement of the target because the RF current automatically flows in the correct direction.

One pre-requisite for an efficient and successful use of said conductive hyperthermia method is a good and **close contact** between the electrodes of the capacitor arrangement and the surface of the target to be treated (e.g. a patient's skin). But a loose contact is not just a problem of efficiency but also poses a risk to the health of a patient (animal or human), since a local heat accumulation can occur in areas of loose contact leading to unpleasant or even harmful burns of the skin. The problem of contact is especially pronounced in conductive hyperthermia treatments, since here treatment sessions usually take at least half an hour and normally one hour and sometimes even longer. Almost unavoidable movements of the patient during that time result in a slipping of the hyperthermia applicator out of position, which can deteriorate the contact between applicator and patient. On the other side, the use of anaesthesia to prevent movements of a patient is also undesirable, because it also poses a burden and risk to the patient's health, especially if hyperthermia treatment is repeated, as it is usually the case, for example if hyperthermia is used concomitant with conventional cancer therapies. Moreover, the problem of contact, as described, is of course more prominent the larger the surface to be treated is, since the larger the surface more and more surface irregularities need to be covered tightly. However, a tumour-cell selective treatment of large body surfaces or body volumes is especially desired, since it would promote the treatment of more spread cancer manifestations such as cancer metastases, which still bear an enormous risk to patients due to their hazardousness in combination with their very limited visibility in diagnostics. Thus, especially for the treatment of cancer metastases and for the after-treatment of large body regions after a cancer therapy a close contact of the RF electrode to the target surface, especially the skin of a patient, is very important. Consequently the objective of the present invention is to provide a RF electrode especially useful for the treatment of large body parts or even the whole body of a target such as a patient, wherein the RF electrode fits to the body shape or is fittable to any body shape in a most optimal manner so that there is in the most optimal case no space between the target-facing side of the RF electrode and the surface of the target such as the skin of a patient.

US2004/044341 discloses conformable electrodes that maintain their shape.

### Description of the invention

The objective is solved by a RF electrode as defined by claim 1, which has a mouldable target-facing side which is fittable or adjustable to any shape of a target such as a patient.

Thus, the present invention is related to a RF electrode or to a bag-like RF electrode with a target-averted side and a mouldable target-facing side and an inner cavity in between, wherein the inner cavity is fillable and emptiable and perfusable with a liquid and / or a liquid substitute and the inner cavity contains solid particles arrangeable in the presence of a liquid and/or a liquid substitute within the inner cavity and at least a part of the surface of the target-facing side is electrically conductive and is connectable to a RF energy source, wherein the target-facing side is fittable to any shape of a target by arranging or moving the solid particles within the inner cavity.

For an optimised RF hyperthermia treatment applying electrically conductive energy transfer, it would be advantageous, if the surface of the electrode of the capacitor, i.e. the surface of the upper electrode which is placed on top of the target, such as a patient, and the surface of the lower electrode (or counter electrode) which is placed below the target is in close and direct contact to the surface of the target, such as the skin of the patient, without any space there between. This allows the optimal electrically conductive energy transfer without the risk of necrosis or burning the skin of the patient.

Therefore the target-facing side of the RF electrode and especially of the upper RF electrode which is placed onto the target (e.g. a patient) should be shape adaptable to the body shape of the target (e.g. of a patient).

The lower RF electrode (the counter electrode), which is placed below the target (e.g. a patient), is preferably a water bed having an electrically conductive surface. Due to the weight of the patient, the water bed will automatically follow the shape of the body of the patient who is lying on the bed. Thus, the problem to have a surface of a RF electrode which follows the shape of the patient without any space between the skin of the patient and the conductive surface of the RF electrode is solved quite easily for the counter electrode which is below the target, i.e. below the patient.

However, for the upper RF electrode on top of the patient (i.e. in form of an overlay), it is a challenge to solve this problem, because a conductive water bed cannot be used due to its weight. It is not acceptable to put a weight of 30 kg, or 20 kg or even 10 kg on the patient during the treatment time of 30 to 60 minutes or even longer. This is inconvenient and also exhaustive for the patient.

Consequently a shape adaptable and light upper RF electrode is required. In case a leg of a patient is treated, the leg has approximately the form of a cylinder with decreasing inner diameter from the buttocks to the foot. Thus the back part of the leg sinks into the water bed (or a different form of lower electrode) which follows the shape of the cylinder. The upper RF electrode has also to follow the shape of the cylinder in order to cover the upper part of the leg. The same is true for an upper RF electrode which should cover the chest of a woman and which has to follow the shape and to provide space for the breasts. Thus, the present invention provides RF electrodes which are adaptable or adjustable or fittable to any body shape. Moreover, in a preferred embodiment such RF electrodes once fitted or adapted to the body shape or to the shape of a body area to be treated are able to keep this shape so that the weight of the RF electrode can be captured by a stand or rack taking over the weight of the electrode, so that the patient does not feel any weight on top of his/her body. In a preferred embodiment of the present invention the inventive RF electrode is fixed to such a rack or stand in a spring-compensated manner to allow a degree of movement and thus retain a higher degree of adaptability.

The present invention is especially directed to the upper RF electrode of a capacitor (or condenser). Said upper RF electrode has a target-facing side which is the bottom side of the electrode and a non-target-facing side or target-averted side which is the upper side or top side of the electrode. The target-averted side is preferably rigid and/or inelastic and gives the general shape to that RF electrode. The target-averted side is preferably formed like a cap or cover or hood over the target-facing side. The target-facing side is preferably almost flat or slightly convex and is made of a flexible, deformable or shape-adaptable material, especially a plastic material.

The RF electrode, and especially the upper RF electrode, has preferably the form of a bag or a mattress with a stiff or rigid target-averted side and a deformable or shape-adaptable target-facing side. The target-facing side together with the target-averted side form a cavity. Thus, between the target-averted side and the target-facing side is a cavity which is filled or partly filled with solid particles. Moreover, the cavity has preferably at least one inlet and at least one outlet for a liquid and/or a liquid substitute so that this cavity between the target-averted side and the target-facing side which contains the solid particles is fillable and emptiable and also perfusable with a liquid, such as water or a water alcohol mixture, and/or a liquid substitute. The solid particles inside the cavity cannot pass the inlet or the outlet for the liquid and/or the liquid substitute and thus remain within the cavity. Moreover, in the case a liquid is used in accordance with invention for partly filling the inner cavity of the RF electrode the solid particles are not soluble or swellable in the liquid and do also not become sticky or gluey in the liquid. Thus, the solid particles are preferably inert in regard to the liquid. The liquid and/or the liquid substitute does also not alter or damage the material of the target-averted side and the target-facing side of the RF electrode. Thus the material of the target-averted side and of the target-facing side is inert in regard to the liquid and/or the liquid substitute.

In case the inventive RF electrode is placed on top of a target (i.e. in form of an overlay), such as the body or a body part of a patient, the target-facing side of the RF electrode assumes the invert form (or shape, contour) of the target by engulfing the target with its flexible or deformable target-facing side. The position of the RF electrode which is preferably the upper RF electrode can now be fixed by a stand or rack so that the weight of the RF electrode lies no longer on the target. Since the target-facing side fits to the shape of the target, there is a tight connection between the target-facing side of the RF electrode and the target, e.g. the skin of a patient. Such tight connection provides the optimal conductive energy transfer from one RF electrode through the target (especially a patient or body part of a patient) to the counter electrode, avoiding burning the patient's skin.

Since after the upper RF electrode was lowered onto the target which is preferably in a horizontal position and its target-facing side assumed the invert shape of the target, the position of the upper RF electrode is fixed by the use of a rack or stand on which the upper RF electrode is arranged or mounted so that there is no weight anymore of the upper RF electrode which lies or pushes on the target. In all embodiments disclosed herein, the target is preferably in a horizontal position and in case of a patient, the patient is preferably lying on a water bed or on an inventive RF electrode.

The target-facing side of the RF electrode will keep the shape assigned by the target to the target-facing side as long as no application of an outer force (i.e. a force that is not exerted by the RF electrode itself) applies, which is able to deform the shape of the target-facing side of the RF electrode. This means, even if the upper RF electrode is removed from the target, the target-facing side will maintain its shape predetermined by the shape of the target onto which the target-facing side of the RF electrode was placed.

Thus, if the patient moves during the RF hyperthermia treatment, the movement is able to generate an outer force which when directed to the target-facing side of the RF electrode is able to deform the shape of the target-facing side so that the shape of the target-facing side is able to follow the amended shape of the patient. This flexibility or deformability of the target-facing side of the RF electrode ensures that also during and after a movement of the patient the close contact of the target-facing side of the RF electrode to the skin of the patient is maintained.

In a preferred embodiment, the shape assigned to the target-facing side of the RF electrode can be stabilized by decreasing the amount of the liquid, such as water, and/or the amount of the liquid substitute present inside the cavity, i.e. present inside the hollow space between the target-averted side and the target-facing side. This stabilizing effect is also called "sand effect". In case a large volume of the liquid and/or of the liquid substitute is present within the inner cavity, the solid particles can move more easily so that the application of a lower force is already sufficient to deform the target-facing side by moving the solid particles inside the inner cavity. In case the amount of liquid and/or the liquid substitute inside the inner cavity is reduced, the solid particles require a larger force to be moved so that a larger force has to be applied against the target-facing side in order to move the solid particles inside the inner cavity. Thus, this effect can be used to stabilize the shape of the target-facing side by reducing the liquid content and/or the liquid substitute content inside the inner cavity or also to make the target-facing side easier deformable by increasing the amount of liquid and/or of liquid substitute inside the inner cavity. The "sand effect" is described in more detail below.

Thus, the present invention is related to a RF electrode or to a bag-like RF electrode or to a mattress-like RF electrode with a stiff or rigid target-averted side and a mouldable or deformable target-facing side and an inner cavity in between, wherein the inner cavity is fillable and emptiable and perfusable with a liquid, such as water, and/or with a liquid substitute and the inner cavity contains solid particles which are inert in the liquid and/or in the liquid substitute and which are arrangeable or movable in the liquid and/or in the liquid substitute within the inner cavity and at least a part of the surface of the target-facing side is electrically conductive and is connectable to a RF energy source, wherein the target-facing side is fittable or adaptable to any shape of a target by arranging or moving the solid particles within the inner cavity.

In other words, the present invention is related to a RF electrode or to a bag-like RF electrode or to a mattress-like RF electrode with a target-averted side and a mouldable or deformable target-facing side and an inner cavity in between both sides, wherein the inner cavity is fillable and emptiable and perfusable with a liquid, such as water, and/or with a liquid substitute and the inner cavity contains solid particles which are inert in the liquid and/or in the liquid substitute and which are arrangeable or movable in the liquid and/or in the liquid substitute within the inner cavity and at least a part of the surface of the target-facing side is electrically conductive and is connectable to a RF energy source, wherein the target-facing side is fittable or adaptable to any shape of a target by arranging or moving the solid particles within the inner cavity.

The present invention is as also directed to a RF electrode or to a bag-like RF electrode or to a mattress-like RF electrode with a target-averted side and a mouldable or deformable target-facing side and an inner cavity in between both sides, wherein the inner cavity is fillable and emptiable and perfusable through at least one inlet and at least one outlet with a liquid, such as water, and/or with a liquid substitute and the inner cavity contains solid particles which are inert in the liquid and/or in the liquid substitute and which are arrangeable or movable in the liquid and/or in the liquid substitute within the inner cavity in order to give any shape to the target-facing side and at least a part of the surface of the target-facing side is electrically conductive and is connectable to a RF energy source, wherein the target-facing side is fittable or adaptable to any shape of a target by arranging or moving the solid particles within the inner cavity by the application of a force or outer force to the target-facing side.

The present invention is also directed to a RF electrode or to a bag-like RF electrode or to a mattress-like RF electrode with a target-averted side and a mouldable or deformable target-facing side and an inner cavity in between both sides, wherein the inner cavity is fillable and emptiable and perfusable with a liquid, such as water, and/or with a liquid substitute and the inner cavity contains solid particles which are inert in the liquid and/or in the liquid substitute and which are arrangeable or movable in the liquid and/or in the liquid substitute within the inner cavity and at least a part of the surface of the target-facing side is electrically conductive and is connectable to a RF energy source, wherein the target-facing side is fittable or adaptable to any shape of a target by arranging or moving the solid particles in the presence of a liquid under application of a force or of an outer force to the target-facing side.

The RF electrode of the present invention is preferably a whole body RF electrode or a large area RF electrode covering the body of a patient or at least large body parts of a patient (e.g. the back, the chest, the entire torso, etc.).

The term **"whole body",** as used herein, is only used when the target to be treated is a living being (i.e. a patient). Herein, the term "whole body" does not refer to the entire surface area of a patient but refers only to the surface area of one side of the patient, those "sides of the patient" being for example the ventral (also referred to as side of the belly), dorsal (also referred to as side of the back), left and right side of the patient's body. Left and right side of the patient's body are understood from the perspective of the patient. Moreover, an inventive RF electrode that covers ≥ 80 % of the surface area of that side of a patient on or under which said RF electrode is placed is referred to as "whole body" RF electrode.

Thus, the present invention is also related to a RF electrode or to a bag-like RF electrode with a target-averted side and a mouldable target-facing side and an inner cavity in between, wherein the inner cavity is fillable and emptiable and perfusable with a liquid and / or a liquid substitute and the inner cavity contains solid particles arrangeable in the presence of a liquid and/or a liquid substitute within the inner cavity and at least a part of the surface of the target-facing side is electrically conductive and is connectable to a RF energy source, wherein the target-facing side is fittable to any shape of a target by arranging or moving the solid particles within the inner cavity, and wherein the RF electrode is a whole body RF electrode.

### Detailed description of the invention

The present invention is related to a radiofrequency electrode (RF electrode), a bag-like radiofrequency electrode (RF electrode) or a bolus radiofrequency electrode with a target-averted side and a mouldable or deformable target-facing side and an inner cavity in between the target-facing side and the target-averted side, wherein the inner cavity is fillable and emptiable and perfusable with a liquid, such as water or a water alcohol mixture, and/or with a liquid substitute and the inner cavity contains solid particles arrangeable or movable in the presence of the liquid and/or the liquid substitute within the inner cavity in order to assume the invert shape of the target so that the target-facing side follows the shape of the target in order to obtain a close and direct contact of the target-facing side to the surface of the target and at least a part of the surface of the target-facing side is electrically conductive and is connectable to a radiofrequency (RF) energy source and the solid particles are arrangeable or movable in the liquid and/or the liquid substitute within the inner cavity by the application of a force to the target-facing side.

The force applied to the target-facing side is on the one side generated by the target (representing a mechanical resistance) and on the other side given by the weight force of the electrode itself, when the RF electrode is lowered onto the target, which is preferably in a horizontal position, so that the target and especially the prominent areas of the target sink(s) into the mouldable or deformable target-facing side thereby arranging or moving the solid particles in the liquid inside the cavity through which the shape assigned to the target-facing side is stabilized.

This stabilization can be explained by the arrangement or movement of the solid particles when a force or outer force is applied against the target-facing side. The shape given to the target-facing side which is the invert shape of the target or target area which is in contact with the target-facing side is conserved even if the application of the force is abandoned. This would not be possible if only a liquid without solid particles is present in the inner cavity, because a liquid filled bag would follow with its target-facing side the shape of a target on which the bag is placed but when removing the bag from the target, the shape of the target-facing side will collapse. However, the degree of stabilization due to the presence of the solid particles within the inner cavity of the inventive RF electrode strongly depends on the ratio between the volume of the liquid and the volume of the solid particles within the inner cavity. In other words, it depends on the density of the solid particles within the inner cavity. This aspect will be elucidated in greater detail below.

However, the assigned invert shape of the contacted target or contacted target area, which is assigned to the target-facing side, is not entirely frozen or rigid but can be deformed again by the application of a force to the target-facing side, however, the amount of force necessary for a deformation depends on the degree of stabilization of the inventive RF electrode. Such a deformation happens, for example, if the target moves under the RF electrode (in form of an upper electrode). After fitting the target-facing side of the upper RF electrode to the shape of the target in order to have a close and direct contact of the electrically conductive surface of the target-facing side to the target (or to the surface of the target), the weight of the upper RF electrode is compensated by a rack or stand so that the target such as a patient does not feel the weight of the upper RF electrode anymore. In a preferred embodiment of the present invention the inventive RF electrode is fixed to such a rack or stand in a spring-compensated manner to allow a degree of movement and thus retain a higher degree of adaptability. However, the fixing of the upper RF electrode to the rack or stand implies the abandonment of a force against the target-facing side which however keeps its shape and does not collapse. If now the target moves under the upper RF electrode, which normally implies that the shape of the target is changed, this change will cause a new force against the target-facing side and will cause adaptation of the shape of the target-facing side to the changed shape (due to the movement) of the target. This new adapted shape of the target-facing side remains even if no further force is applied as it is the case after the movement of the target is stopped. Thus, the target-facing side of the RF electrode follows the shape of the target or target area, which is in contact with the target-facing side, also in case the target moves and changes its shape due to the movement.

The term **"target",** as used herein, refers to the body of the object to be treated, which can be a living being, such as a human or an animal, but it can also be a non-living structure, such as for instance a container filled with physiological saline. "Target" and "target body" are used synonymously in this application. In the case, that the "target" is a living being, it is also sometimes referred to the "target" as **"patient".**

The term **"liquid",** as used herein, refers to a substance or mixture of substances that at standard ambient temperature and pressure (SATP) conditions (temperature 25 °C; pressure 1013 hPa) is in the liquid state of matter, which means that it can flow, is essentially non-compressible and has a defined volume but not a defined shape, i.e. it adapts to the shape of its container but does not expand to fill this container. Clearly delimited hereof is the term **"fluid",** which refers both to substances, which are in the liquid state of matter, as well as to substances, which are in the gaseous state of matter.

The term **"solid",** as used herein, refers to a body that at standard ambient temperature and pressure (SATP) conditions (temperature 25 °C; pressure 1013 hPa) is in the solid state of matter, thus the forces between the molecules forming the body are strong so that the particles preferably cannot move freely but can only vibrate. As a result, a solid has a stable, definite shape, and a definite volume at a given temperature and pressure.

The term **"solid particles",** as used herein, refers to particles that are in the solid state of matter and that are not able to pass through the at least one inlet or the at least one outlet of said inventive RF electrode and are hence remaining within the inner cavity of the inventive RF electrode during the operation of the device. Thus, said "solid particles" are sometime also referred to as **"caged particles".**

The term **"liquid substitute",** as used herein, refers to a liquid-like substance formed by a multitude of particles that are in the solid state of matter but that behave like a liquid. Hence, the particles forming the "liquid substitute" are sometimes also referred to as **"liquid-like particles".** Due to their low friction they are easily sliding against each other and against other materials and are thus flowing quite similar to a real liquid. In addition, as used herein, the "liquid substitute" (in contrast to the "solid particles") is able to pass through the at least one inlet and the at least one outlet of said inventive RF electrode.

The term **"mouldable",** as used herein, means that the respective device is flexible and can be deformed.

The term **"target-facing side",** as used herein, refers to the side of the inventive RF electrode that is in opposite to and directed towards the surface of the target.
The term **"surface of the target-facing side",** as used herein, refers to the surface of said target-facing side that is designed to be in direct physical contact to the surface of the target. Thus, the surface of said target-facing side can come into contact with the target while the "contact face of the target-facing surface" is the part of the surface of the target-facing side which is actually in contact with the target. The part of the surface of the target-facing side that is electrically conductive is sometimes also referred to as **"conductive layer".** In contrast, the term **"target non-facing side"** or **"target-averted** side", used synonymously in the present application, refer to the side of the inventive RF electrode that is directed away from the surface of the target or separated from it at least by the target-facing side of the inventive RF electrode. The unit of target-facing side and target-averted side optionally together with a side part of the inventive RF electrode are forming a so called **"envelope"** (also called sheath) enclosing the inner cavity of the inventive RF electrode. Said "side part" is connecting the target-averted and the target-facing side of the inventive RF electrode to close the envelope that encases the inner cavity. However, in other embodiments of the invention, it is also conceivable that the margins of the target-facing side and the target-averted side are directly merging and form alone the continuous envelope of the inner cavity, without a need of a side part.

Thus, the present invention is also directed to a RF electrode with a target-averted side and a mouldable target-facing side and a side part connecting the target-averted side and the mouldable target-facing side to form an inner cavity in between, wherein the inner cavity is fillable and emptiable and perfusable with a liquid and/or a liquid substitute and the inner cavity contains solid particles arrangeable in the presence of a liquid and/or a liquid substitute within the inner cavity and at least a part of the surface of the target-facing side is electrically conductive and is connectable to a RF energy source, wherein the target-facing side is fittable to any shape of a target by arranging the solid particles within the inner cavity.

The term **"fittable",** as used herein, means that the respective device or a part thereof can be adapted to the shape (or contours) of a target, on which the device or a part thereof is placed. This term does not imply, however, whether the device or a part thereof retains the shape it has been adapted to or whether it regains its initial shape once being detached from the target. The terms "fittable" and "shape-adaptive" are used synonymously in this application.

It shall be noted here, that when a device or part thereof, such as the target-facing side of the inventive RF electrode, is fitted to a target's shape it adopts the inverted shape of the target. As a result, the fitted device (e.g. RF electrode) is able to preferably closely cover the surface of the target such as for instance in the field of handicraft a casted object fits perfectly into its corresponding mould.

In a preferred embodiment of the present invention, the inventive RF electrode is placed on top of the target to be treated, i.e. in form of an overlay that covers the target or at least parts thereof. In this special embodiment and especially if the target is a patient (human or animal) it is desirable that the inventive RF electrode is as light-weight as possible in order to minimize the burden to the target (e.g. the patient).

Thus, the present invention is also related to a RF electrode in form of an overlay with a target non-facing side and a mouldable target-facing side and an inner cavity in between, wherein the inner cavity is fillable and emptiable and perfusable with a liquid and/or a liquid substitute and the inner cavity contains solid particles arrangeable in the presence of a liquid and/or a liquid substitute within the inner cavity in order to give any shape to the target-facing side and at least a part of the surface of the target-facing side is electrically conductive and is connectable to a RF energy source, wherein the target-facing side is fittable to any shape of a target by arranging the solid particles in the presence of a liquid and/or a liquid substitute under application of a force.

The term **"in form of an overlay",** as used herein, refers to an embodiment of the present invention, wherein the inventive RF electrode is designed to be placed on top of the target. In embodiments of the present invention, in which the inventive RF electrode is used in form of an overlay, the target-facing side of the inventive RF electrode is the bottom side of the RF electrode, i.e. the target-facing side of the RF electrode is oriented in direction of the gravitational force so that the gravitational force of the solid particles inside the inner cavity acts on the inner surface (i.e. inside the inner cavity) of the target-facing side.

Thus, the present invention is also directed to an RF electrode with a target-averted side and a mouldable target-facing side and an inner cavity in between, wherein the inner cavity is fillable and emptiable and perfusable with a liquid and/or a liquid substitute and the inner cavity contains solid particles arrangeable in the presence of a liquid and/or of a liquid substitute within the inner cavity and at least a part of the surface of the target-facing side is electrically conductive and is connectable to an RF energy source, wherein the target-facing side is fittable to any shape of a target by arranging the solid particles within the inner cavity, and wherein the RF electrode is in form of an overlay placeable on top of the target.

In other words, the present invention is also directed to an RF electrode with a target-averted side and a mouldable target-facing side and an inner cavity in between, wherein the inner cavity is fillable and emptiable and perfusable with a liquid and/or a liquid substitute and the inner cavity contains solid particles arrangeable in the presence of a liquid and/or of a liquid substitute within the inner cavity and at least a part of the surface of the target-facing side is electrically conductive and is connectable to a RF energy source, wherein the target-facing side is fittable to any shape of a target by arranging the solid particles within the inner cavity, wherein the RF electrode is in form of an overlay placeable on top of the target, characterized in that the target-facing side is the bottom side of the RF electrode and that the gravitational force of the solid particles inside the inner cavity acts on the inner surface (i.e. inside the inner cavity) of the target-facing side and is directed to the target.

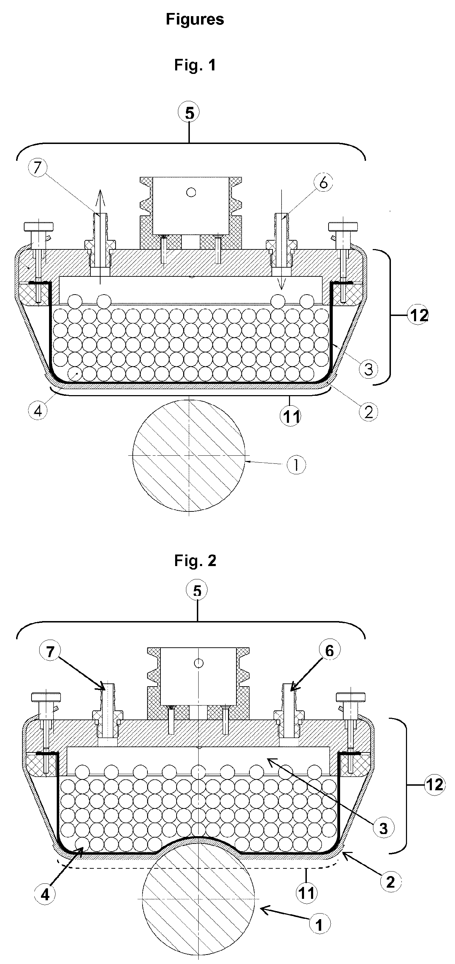

The spatial orientation of the inventive RF electrode is important. The target-facing side is the bottom side of the RF electrode as shown in Figure 1 and the surface of the target-facing side is in the initial and not deformed or not moulded state most preferably a horizontal plane as shown in Figure 1. The initial state refers to the shape of the surface of the target-facing side before the electrode is placed on the target. Thus the RF electrode is spatially oriented so that the gravitational force of the solid particles is directed to the inner surface of the target-facing side. The inner surface of the target-facing side is the surface of the target-facing side which is inside the inner cavity.

It is preferred according to the present invention, that the inner cavity of the inventive RF electrode contains a liquid and/or a liquid substitute at least in the operational state of the inventive device. Thus, the present invention is also related to a RF electrode with a target-averted side and a mouldable target-facing side and an inner cavity in between, wherein the inner cavity contains a liquid and/or a liquid substitute as well as solid particles arrangeable in the liquid and/or the liquid substitute within the inner cavity,
wherein the inner cavity is perfusable with said liquid and/or liquid substitute,
and wherein at least a part of the surface of the target-facing side is electrically conductive and is connectable to a RF energy source,
wherein the target facing side is fittable to any shape of a target by arranging the solid particles in the liquid under application of a force.

The liquid and the solid particles should have the properties as disclosed herein also for this embodiment like being inert, not soluble, not sticky and so on.

Thus, the present invention is also related to an RF electrode with a target-averted side and a mouldable or deformable target-facing side and an inner cavity in between, wherein the inner cavity is fillable and emptiable and perfusable with a liquid and/or a liquid substitute and the inner cavity contains solid particles arrangeable or movable in the presence of a liquid and/or a liquid substitute within the inner cavity and at least a part of the surface of the target-facing side is electrically conductive and is connectable to a RF energy source, wherein the solid particles are loose particles which do not adhere or stick to each other, which are neither electrically conductive nor magnetic and if a liquid is present also not soluble in the liquid.

### Envelope & Cavity

The present invention is related to an RF electrode comprising a target-facing and a target-averted side enclosing an inner cavity filled with solid particles and at least in the operational state also with a liquid and/or a liquid substitute. Hence, it is preferred that the inner cavity is not completely filled with the solid particles. Thus, the inner cavity is preferably only partly filled with the solid particles. More preferably the totalized volume **(Vp** - see further below for definition) of all solid particles (also referred to as caged particles) within the inner cavity is less than 52.4%, more preferably less than 50.0%, more preferably less than 45.0%, more preferably less than 40.0%, more preferably less than 35.0%, more preferably less than 30.0%, more preferably less than 25.0%, more preferably 20.0% of the total inner volume of the inner cavity (Vₜₒₜₐₗ - see further below for definition) in the state, in which the inventive RF electrode is not adapted to a target (e.g. a patient). In other words, the totalized volume (**V**_{P}) of all solid particles (also referred to as caged particles) within the inner cavity is preferably between 20.0% and 52.4%, preferably between 25.0% and 50.0%, more preferably between 30% and 45% of the total inner volume of the inner cavity (Vₜₒₜₐₗ) in the state, in which the inventive RF electrode is not adapted to a target (e.g. a patient). It shall be mentioned here, that the total inner volume of the inner cavity (Vₜₒₜₐₗ) is not constant during the operation of the inventive RF electrode. During fitting of the RF electrode (cf. Fig. 1 to 3) to the shape of the target (e.g. a patient) the volume of the liquid and/or liquid substitute within the cavity can be reduced on purpose, this goes along with a reduction of the total inner volume of the inner cavity (Vₜₒₜₐₗ) and with an increasing density of the solid particles within the inner cavity. Thus, in preferred embodiments of the present invention the percentage of the inner cavity's volume occupied by the solid particles may change from low percentage values to higher percentage values, given above. In this context it shall be noted that the volume of liquid and/or liquid substitute withdrawn from the inner cavity during the fitting to a target (e.g. a patient) does preferably not exceed an extent, that not enough liquid (and/or liquid substitute) is left to fill the interstices between the solid particles. This is not only necessary to ensure a continuous low flow of the liquid and/or the liquid substitute e.g. for temperature-control during the hyperthermia treatment but also to allow a certain degree of adaptability during the hyperthermia treatment. Thus, the total inner volume of the inner cavity (Vₜₒₜₐₗ) shall only be reduced to a maximal extent that the total inner volume of the inner cavity (Vₜₒₜₐₗ) is still at least 191% of the totalized volume of all solid particles (V_{P}) within the inner cavity. It is further preferred in accordance with the present invention, that the number of the solid particles inside the inner cavity is sufficient to cover the surface of the target facing-side of the inventive RF electrode at least in a double layer, preferably at least a triple layer, even more preferably at least in 4 layers, more preferably at least in 5 layers, even more preferably at least in 8 layers and still more preferably at least in 10 layers. Dependent on the size of the solid particles used, it is also conceivable in certain embodiments of the present invention, especially when the solid particles are very small in size, that the number of solid particles used in the respective embodiment is sufficient to cover the target facing-side of the inventive RF electrode at least in 100 or 1000 or even more layers. In consequence, it is obvious to a person skilled in the art, that the width of the inner cavity above the surface of the target-facing side has to be large enough to accommodate the at least double layer of the solid particles used, preferably the at least triple layer, or the at least 4 layers, or the at least 5 layers, or the at least 8 layers, or the at least 10 layers, or the at least 100 layers or the at least 1000 layers of the solid particles used. However, the entire dimensions of the inner cavity shall preferably not lead to a total inner volume of the inner cavity (Vₜₒₜₐₗ) in the initial state, where the inventive RF electrode is not adapted to a target, that it is out of the range 5.0 times - 2.0 times, preferably 4.5 times - 2.5 times of the totalized volume of all solid particles (**V**_{P}) used in the respective embodiment.

The **"width of the inner cavity"** shall be explained with an idealized embodiment of an inventive RF electrode, which has a perfectly rectangular shape. The width of the inner cavity is the distance between the target-facing side and the target-averted side of the inventive RF electrode that are both oriented horizontally and perfectly parallel to each other. However, a person skilled in the art is aware that the situation is more complex in reality, since, first, the RF electrode might not be a perfect rectangle but might for example have a convex target-facing side and, second, during the fitting process of the inventive device the target-facing side is adapted to the contours of a target and has thus a varying width. In consequence, the width of the inner cavity, used herein, usually refers to the average width of the inner cavity during the non-adapted state of the inventive RF electrode.

The inventive electrode resembles a bag and is therefore also referred to as bag-like RF electrode.

According to the present invention, the target-facing side of the bag-like RF electrode shall be mouldable in order to be able to adapt to the surface of the target. This serves on the one hand to establish the essential close contact between the target-facing surface of the RF electrode and the surface of the target. On the other hand, it also enables a secure positioning of the inventive device on the target during the course of the treatment, since it prevents the inventive device to easily slip out of position. Thus, the inventive RF electrode or bag-like RF electrode comprises a target-facing and a target-averted side, which together (and optionally with a side part) form an envelope enclosing the inner cavity of the inventive RF electrode. At least in the operational state of the inventive RF electrode, said inner cavity is filled with solid particles as well as a liquid and/or a liquid substitute.

Thus, the material of the target-facing side of the RF electrode needs to be not only **flexible** but also **leak-proof** to the liquid (and/or the liquid substitute) used in the respective embodiment of the inventive device. Possible materials for the target-facing side of the inventive RF electrode comprise: soft polyvinyl chloride (PVC), polyvinylidene chloride (PVdC), polyethylene (PE), polypropylene (PP), polyurethane (PU), and silicone.

In a further embodiment of the invention, the inner cavity of the RF electrode has at least one inlet and at least one outlet to permit the influx and efflux of the liquid and/or the liquid substitute used during RF hyperthermia treatment into and from said inner cavity. In a preferred embodiment of the present invention means are provided within the at least one inlet and/or the at least one outlet to prevent the solid particles contained in said inner cavity from passing through the at least one inlet and/or the at least one outlet. Such means can comprise nets, meshes, grids, filters and the like.

In a further embodiment of the invention, the inner cavity of the RF electrode is divided into several separate cavities that are connected to each other forming a continuous volume. Alternatively the inner cavity of the inventive RF electrode may be divided into several separate cavities that are not connected to each other, thus having each at least one inlet and at least one outlet. It is also conceivable that the inner cavity of the inventive RF electrode is divided into several separate cavities, wherein one fraction of said separate cavities is connected to each other having at least one common inlet and at least one common outlet; wherein the other fraction of said separate cavities is not connected to each other and each of these cavities having thus at least one inlet and at least one outlet.

Thus, in a further embodiment of the present invention, the inner cavity of the RF electrode can be divided into several separate cavities connected to each other or not connected to each other having separate inlets and outlets for the liquid and/or the liquid substitute.

It shall be stated here that the terms **"outlet"** and **"inlet"** refer to ports that connect the inner cavity of the inventive RF electrode to the outside, e.g. to a pumping circuit, and does not refer to connections between separate cavities within the envelope of the inventive RF electrode. Additionally, it shall be pointed out that the "outlet" and "inlet" do not necessarily have to meet the same criteria regarding flexibility of the material than the target-facing side of the inventive RF electrode, since they are usually located on the target-averted side of the inventive RF electrode.

In accordance with the present invention, the target-averted side of the inventive RF electrode or bag-like RF electrode does not need to have the same physical and/or chemical properties than the target-facing side of the inventive bag-like RF electrode.

In contrast to the target-facing side, the target-averted side of the inventive RF electrode or bag-like RF electrode can be rigid or stiff or not deformable in accordance to a preferred embodiment of the present invention.

There are various ways to realize differences in physical and/or chemical properties of the target-facing side compared to the target-averted side of the inventive bag-like RF electrode.

In one embodiment, the target-facing and the target-averted side of the inventive RF electrode can be composed of different materials or mixtures of materials having different material properties but being tightly connected to each other at the junction of the target-facing and the target-averted side (or connected to each other via a connecting side part). In this case, although having different mechanical properties regarding flexibility, the material or mixture of materials of both the target-facing and the target-averted side of the inventive bag-like electrode need to be leak-proof to the liquid and/or the liquid substitute used in accordance with the invention as filling of the inner cavity.

In another embodiment, the envelope encasing the inner cavity can be composed entirely of a material or mixture of materials having the properties desired for the target-facing side of the inventive bag-like RF electrode. Then, the target-averted side of this envelope can be attached to and thereby supported by an overlying material layer (e.g. a cap) that is rigid and thus confers rigidity to the target-averted side of the envelope. Such an attachment can for example be made irreversible by means of gluing or welding or detachable by means of straps, snap buttons, hook and loop fasteners (Velcro^{®} fasteners), zip fasteners and the like. A person skilled in the art is aware of other solutions for such an attachment that can be used in accordance with the invention.

In a preferred embodiment of the present invention, the target-averted side of the RF electrode is rigid and has the following properties:
(1) it defines and stabilizes a general shape of the inventive RF electrode,
(2) it allows the stable and reliable attachment of components being part of the inventive RF electrode (e.g. ports as inlet and/or outlet to the inner cavity),
(3) it additionally allows the connection to components different from the inventive RF electrode (e.g. a swivel arm, pumping circuit, etc.) but being part for instance of the hyperthermia device the inventive RF electrode is incorporated into.

These points listed above can be especially helpful with respect to a safe and reliable positioning of the RF electrode and also if a fast retraction of the inventive RF electrode is necessary due to an emergency situation.

Thus, a preferred embodiment of the present invention is related to a RF electrode with a rigid or non-deformable or stiff target-averted side and a mouldable or deformable target-facing side and an inner cavity in between, wherein the inner cavity is fillable and emptiable and perfusable with a liquid and/or with a liquid substitute and the inner cavity contains solid particles arrangeable or moveable in the presence of a liquid and/or of a liquid substitute within the inner cavity in order to give any shape to the target-facing side and at least a part of the surface of the target-facing side is electrically conductive and is connectable to a RF energy source.

Concerning the overall shape of the inventive RF electrode, the target-facing side and the target-averted side viewed from below (i.e. viewed from the perspective of the surface to be treated) there is no limitation. This means, the inventive RF electrode and/or the target-facing side of the inventive RF electrode and/or the target-averted side of the inventive RF electrode can be round, oval, polygonal such as trigonal, tetragonal (e.g. square, rectangular, trapezoid), pentagonal, hexagonal, heptagonal, octagonal, and so on, when viewed from below. Moreover, in case of a polygonal shape, the polygon can also have rounded edges or truncated edges or a mixture of both. The person skilled in the art is aware of other shapes that can be realized in the present invention.

In some embodiments of the present invention the overall shapes of the target-facing side of the inventive RF electrode and of the target-averted side of the inventive RF electrode are similar (e.g. both are round or both are rectangular and so on).

According to other embodiments of the present invention, it is also conceivable that the target-facing side of the inventive RF electrode and the target-averted side of the inventive RF electrode have different shapes when viewed from below (i.e. viewed from the perspective of the surface to be treated). This is especially valid in embodiments, in which the target-facing side of the inventive RF electrode and/or the target-averted side of the inventive RF electrode are made of different materials and/or when the target-facing side of the inventive RF electrode has a different diameter than the target-averted side of the inventive RF electrode. For example, in one embodiment of the invention the diameter of the target-facing side of the inventive RF electrode is smaller than the diameter of the target-averted side of the inventive RF electrode. If the target-facing side of the inventive RF electrode has a smaller diameter than the target-averted side of the inventive RF electrode, it is clear that the shape of the target-facing side of the inventive RF electrode can be viewed best from below and not from the top view. In another embodiment of the invention the diameter of the target-facing side of the inventive RF electrode is larger than the diameter of the target-averted side of the inventive RF electrode. If the target-facing side of the inventive RF electrode has a larger diameter than the target-averted side of the inventive RF electrode, it is clear that the shape of the target-averted side of the inventive RF electrode can be viewed best from the top view and not from below. It is for example possible that the target-averted side of the inventive RF electrode has a square overall shape, while the target-facing side of the inventive RF electrode has a round overall shape.

It is evident, that the overall shape of the inventive RF electrode and/or the target-facing side and/or the target-averted side from top view or viewed from below strongly depend(s) on the specific target to be treated. Since it is the target-facing side of the inventive electrode that is brought into contact with the target during treatment, the surface of the target to be treated that is covered by the target-facing side of the inventive electrode corresponds in shape to the shape of the target-facing side of the inventive electrode as delineated above, i.e. round, oval, polygonal such as trigonal, tetragonal (e.g. square, rectangular, trapezoid), pentagonal, hexagonal, heptagonal, octagonal, and so on.

The term "overall shape", as used herein, refers to the two-dimensional contours of an object (here for example the RF electrode, its target-facing side or its target-averted side) when viewed from a certain perspective as specified, e.g. from below (i.e. as viewed from the perspective of the surface to be treated) or from top view.

As evident from the above description, the present invention can be realized with both a liquid and a liquid-like component (liquid substitute) as further filling material of the inner cavity in addition to the solid particles. However, since some technical adaptations are necessary dependent on whether a real liquid or a liquid substitute is used together with the solid particles, the following part of the description is divided into two main chapters for clarity reasons, dealing either with embodiments having solid particles and liquid within the inner cavity (next chapter) or with embodiments having solid particles and liquid substitute within the inner cavity of the inventive RF electrode (next but one chapter).

### Embodiments - Solid Particles and Liquid within the Inner Cavity

### Liquid

In accordance with the invention, the **liquid** can be a non-viscous liquid, a gel, oil or a mixture thereof. The liquid has several functions. The perfusion of the liquid through the inner cavity can be used for temperature-controlling purposes, i.e. cooling or heating the RF electrode and thus the target, such as the skin of the patient, and for supporting the arrangement or move of the solid particles as well as stabilizing the shape assigned to the target-facing side by stabilizing the position and arrangement of the solid particles depending on the volume of liquid present in the inner cavity (see also "sand effect"). Consequently the amount or volume of the liquid present in the inner cavity during the hyperthermia treatment session is normally not constant.

In a preferred embodiment of the present invention, the liquid filling the inner cavity of the inventive RF electrode is water or a mixture of water and ethanol or a mixture of water and isopropanol.

It shall be mentioned that a person skilled in the art is aware that the liquid filling partly the inner cavity may contain unavoidable amounts of gases (e.g. air), which are dissolved in the liquid.

Apart from water, other liquids are conceivable as the liquid filling the inner cavity of the inventive RF electrode. Thus, the liquid suitable to be used in the inner cavity of the RF electrode can be selected from the group of water, ethanol, iso-propanol and mixtures thereof. A person skilled in the art will recognize other liquids suitable for the use in the inventive RF electrode.

Another embodiment of the present invention is related to a RF electrode with a target-averted side and a mouldable target-facing side and an inner cavity in between, wherein the inner cavity is fillable and emptiable and perfusable with a liquid and the inner cavity contains solid particles arrangeable or movable in the presence of a liquid within the inner cavity and at least a part of the surface of the target-facing side is electrically conductive and is connectable to a RF energy source, and wherein the liquid is water, ethanol, iso-propanol or mixtures thereof.

Preferably the liquid consists of at least 60 % by volume water, more preferred of at least 70 % by volume water, even more preferred of at least 80 % by volume water, and most preferred of at least 90 % by volume water.

It is also conceivable to add substances ("additives") to the liquid filling the inner cavity of the inventive RF electrode in order to preserve the liquid non-perishable, for example by preventing bacterial or fungal growth.

The term **"additives",** as used herein, generally refers to substances that are added to the liquid filling the inner cavity of the inventive RF electrode in a volume fraction of less than 10 %, preferably less than 8%, more preferably less than 6%, more preferably less than 4%, and even more preferably less than 2%.

Thus, another aspect of the present invention is related to an embodiment of the present invention, wherein the liquid and preferably water or a mixture of water and ethanol or a mixture of water and iso-propanol further contains at least one anti-fungal, anti-bacterial, antiviral and/or anti-algae additive.

Yet another aspect of the present invention is to use gliding materials (e.g. oil dispersion, MoS₂, etc.) as additives to the liquid to ensure an easy and sufficient gliding of the solid particles filling the inner cavity of the inventive RF electrode with respect to each other or with respect to the walls of the envelope.

However, in case any additives are used, it is preferred to use additives in a concentration which is essentially non-toxic to humans or animals to avoid any harm to a patient in the event of an accidental spill.

### Solid Particles

Another important aspect of the present invention is related to the **solid particles** (also referred to as caged particles) filling at least partly the inner cavity of the inventive RF electrode. The solid particles filling the inner cavity of the inventive RF electrode can be composed of various materials and be present in various shapes and sizes. However, said solid particles (also caged particles) are not able to pass through the at least one inlet or the at least one outlet of the inner cavity of the inventive RF electrode. Thus the number of solid particles remains constant within the inner cavity during the operation of the inventive device.

Hence in accordance with certain embodiments of the invention, it is preferred to place a filter to both pumping circuit ports (inlet, outlet) to avoid obstruction caused by the solid particles.

In a preferred embodiment of the present invention, the solid particles as a part of the filling of the inner cavity of the inventive RF electrode shall meet the following criteria regarding material properties:
(1) They are insoluble in the liquid filling the inner cavity used in accordance with the invention,
(2) they are not electrically conductive,
(3) they are not magnetic,
(4) they do not stick or adhere together, especially in a wet environment, and do not form aggregates, i.e. they are loose particles,
(5) they are not swellable in the liquid used,
(6) they keep their shape under the hyperthermia treatment conditions,
(7) they do not change their physical and/or chemical properties (e.g. shape, state of matter) fundamentally in the temperature and pressure range applied according to the invention,

The solid particles have preferably a density below 2.4 g/cm³, preferably a density below 2.2 g/cm³, more preferably a density below 2.0 g/cm³, preferably a density below 1.5 g/cm³, preferably a density below 1.2 g/cm³, preferably a density below 1.1 g/cm³, preferably a density below 1.0 g/cm³, preferably a density below 0.9 g/cm³, preferably a density below 0.8 g/cm³, preferably a density below 0.7 g/cm³, preferably a density below 0.6 g/cm³, preferably a density below 0.5 g/cm³, and preferably a density below 0.4 g/cm³. It is more preferred that the solid particles have a density in the range of the density of the liquid, i.e. that the solid particles have a density that deviates not more than ± 10%, preferably not more than ±8%, more preferably not more than ±6%, still more preferably not more than ±4% and most preferably not more than ±2% from the density of the liquid. In case the liquid is a mixture of liquids, such as water : ethanol (80 vol% : 20 vol%), the density refers to the density of the mixture. It is further preferred, if the solid particles have a density between 0.4 g/cm³ - 2.4 g/cm³, more preferably a density between 0.5 g/cm³ - 2.0 g/cm³, more preferably a density between 0.6 g/cm³ - 1.6 g/cm³, yet more preferably a density between 0.7 g/cm³ - 1.4 g/cm³, more preferably a density between 0.8 g/cm³ - 1.2 g/cm³, even more preferably a density between 0.9 g/cm³ -1.1 g/cm³.

It is especially preferred in accordance with the invention, if the solid particles as a part of the filling of the inventive RF electrode's inner cavity meet all seven criteria listed above.

Thus, the present invention is also related to a RF electrode with a target-averted side and a mouldable or deformable target-facing side and an inner cavity in between, wherein the inner cavity is fillable and emptiable and perfusable with a liquid and the inner cavity contains solid particles arrangeable or movable in the presence of a liquid within the inner cavity and at least a part of the surface of the target-facing side is electrically conductive and is connectable to a RF energy source, and the solid particles are loose particles and not soluble in the liquid and neither electrically conductive nor magnetic.

Potential materials the solid particles filling at least partly the inner cavity of the inventive RF electrode can be made of comprise: polyurethane (PU), polytetrafluoroethylene (PTFE, Teflon^{®}), fluorinated ethylene propylene (FEP), perfluoroalkoxy polymer (PFA), poly(methyl methacrylate) (PMMA), polycarbonate (PC), polyamide (PA), polyvinyl chloride (PVC), polyoxymethylene (POM), polyethylene (PE), polypropylene (PP), polymethylpentene (PMP), polystyrene (PS) and mixtures thereof. A person skilled in the art is aware of additional materials that fulfil at least most of the above-mentioned criteria and thus can be used in accordance with the invention. Furthermore, it is desired in accordance with the invention, if the material of the solid particles at least partly filling the inner cavity of the inventive RF electrode allows a smooth gliding of said particles against each other (i.e. low friction) in order to promote the adaption to the shape of the target. Of course, it is also desirable in accordance with the invention, if the solid particles are made of a material that is also biocompatible, i.e. non-toxic to humans or animals, in order to avoid any harm to a patient in the event of an accidental spill.

In certain embodiments of the present invention, it is advantageous, if the solid particles at least partly filling the inner cavity of the inventive RF electrode are made from a material that has a lower physical density than the liquid filling the inner cavity of the inventive RF electrode. This helps in reducing the weight of the inventive device and is especially preferred, if the inventive RF electrode is used as an overlay, i.e. is placed on top of the target object.

It is conceivable in accordance with the present invention that the solid particles at least partly filling the inner cavity of the inventive RF electrode can be present in various shapes and sizes.

Possible shapes of the solid particles comprise spheres, rectangles, triangles, and polygons of symmetrical or asymmetrical conformation, but are not limited thereupon. Of course, the solid particles shall have no sharp edges that could pierce the envelope of the inner cavity or that could elicit an unpleasant sensation in a patient. A person skilled in the art is aware of additional shapes that are suitable in accordance with the invention.

However, an important aspect of the present invention regarding the solid particles at least partly filling the inner cavity of the inventive RF electrode is that the solid particles permit a variable but continuous flow of the liquid through the inner cavity of the inventive device during the entire treatment time. As described below in more detail, shape adaption of the inventive RF electrode is facilitated by a purposeful reduction of the liquid's volume inside the inner cavity, while the totalized volume of all the solid particles inside the inner cavity remains constant. Hence, the packaging of the solid particles inside the inner cavity gets denser during shape adaptation. In order to ensure a continuous flow of the liquid it is thus important that interstices between the solid particles persist even after the packaging of said particles gets denser. Hence, it is favourable if the solid particles have a shape that does not tightly interlock, such as for example a spherical shape which is preferred.

The size of the solid particles depends, of course, on the size of the whole RF electrode and also on the volume of the inner cavity. However, for all embodiments it can be stated that the solid particles should preferably not have a diameter below 0.05 mm, preferably below 0.10 mm, preferably below 0.20 mm, more preferably below 0.50 mm, still more preferably below 1.00 mm, more preferably below 2.00 mm and preferably below 3.00 mm, more preferably below 4.00 mm and most preferably below 5.00 mm. In addition, the solid particles should not have a diameter exceeding 10.00 mm, preferably exceeding 12.00 mm, preferably exceeding 14.00 mm, preferably exceeding 16.00 mm, preferably exceeding 18.00 mm, preferably exceeding 20.00 mm. Of course, the values for the diameter of the solid particles, given above, preferably refer to solid particles with a spherical shape.

It shall be understood that the values for the sphere diameter are average diameters, since one cannot exclude minor irregularities in the shape of the spheres, i.e. one cannot exclude that the spheres are not perfectly round or not perfectly identical to each other. However, the standard deviation from this average value for the sphere diameter shall not exceed ±20.0%, preferably not ±10.0% and most preferably not ±5.0% of the average value.

The term **"treatment time",** as used herein, refers to the period of time, that follows after the correct placement of the inventive RF electrode and the shape adaptation and in which the target is subjected to the actual RF hyperthermia treatment.

Such a spherical shape of the solid particles at least partly filling the inner cavity of the inventive RF electrode is also favourable with respect to another technical difficulty arising from the packaging of said particles getting denser in the course of shape adaptation: If the solid particles get into closer contact after reduction of the liquid's volume within the inner cavity and the concomitant reduction of the total inner volume of the inner cavity, they might cant depending on shape. Such a cant might lead to unwanted changes of overall shape of the target-facing side of inventive RF electrode.

Thus, in a preferred embodiment of the present invention, the solid particles at least partly filling the inner cavity of the inventive RF electrode have a spherical shape.

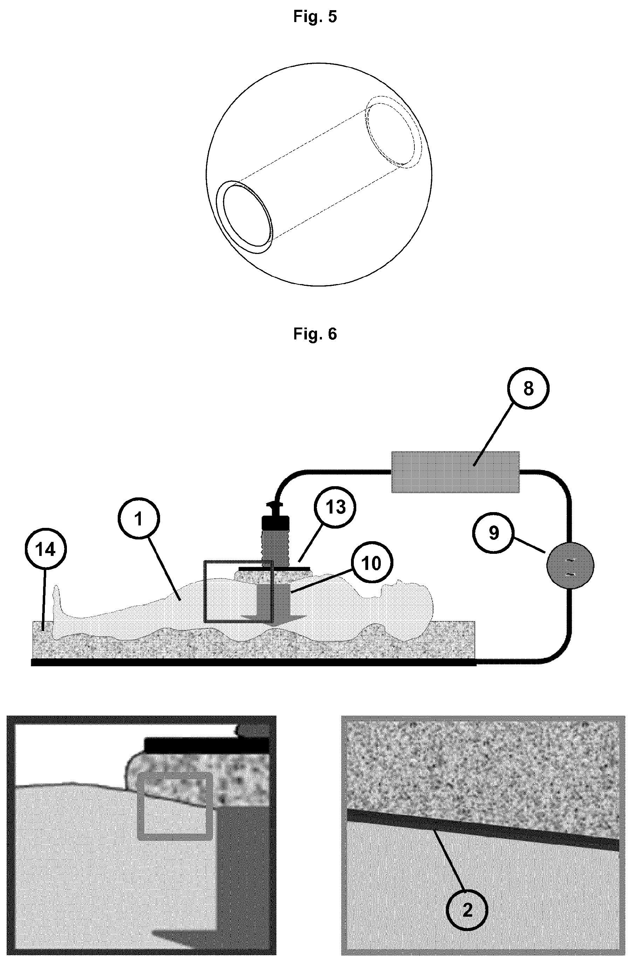

In another embodiment of the present invention, holes can be drilled through the solid particles (cf. Fig. 5) to further facilitate the continuous flow of the liquid during the treatment time. However, care shall be taken, that such holes do not jeopardize the stability of said solid particles, so that they do not collapse or break during the operation of the inventive device.

Another aspect of the present invention is related to the size of the solid particles at least partly filling the inner cavity of the inventive RF electrode. There exists an important trade-off that needs to be considered when choosing the size of the solid particles at least partly filling the inner cavity of the inventive RF electrode: The smaller the particles are the smoother is the adaptation to the contours of the target but also the smaller are the interstices between said particles. The latter might impair the flow of the liquid inside the inner cavity of the inventive RF electrode. In turn, the larger the particles are the rougher is the adaptation to the target but the larger are the interstices between the particles. Hence, large particles might facilitate the flow of the liquid inside the inner cavity of the inventive RF electrode, but can impair the adaptation and might in addition elicit an unpleasant sensation of the individual particles in case a living being (human or animal) is the target to be treated. Thus, the choice of particle size shall be made to ensure a sufficient adaptation of the target-facing side of the inventive RF electrode to the shape of the target, while at same time allowing a continuous flow of the liquid inside the inner cavity during the treatment time. Additionally, in case the inventive device is used on a patient (human or animal) an unpleasant sensation due to perception of the particles as such shall be prevented. Especially the latter aspect strongly depends also on the material and thickness of the target-facing side of the RF electrode. The thickness of the target-facing side together with the diameter of the solid particles shall be chosen, so that the target-facing side of the inventive RF electrode can adapt smoothly to the surface of the target (e.g. the patient's skin), wherein smooth means that the individual solid particles do not produce indentations in the target-facing side that are perceivable by the patient. In consequence, it is obvious for a person skilled in the art that an optimal particle size depends on the intended application or the precise embodiment of the inventive RF electrode and thus needs to be determined experimentally. However in another embodiment of the present invention, the thickness of the target-facing side is preferably between 0.5 mm - 10.0 mm, still more preferably between 1.0 mm - 8.0 mm, more preferably between 1.5 mm - 7.0 mm, even more preferably between 2.0 mm - 6.0 mm, more preferably between 2.5 mm - 5.0 mm and especially preferred between 3.0 mm - 4.0 mm.

It shall be understood that the values given for the thickness of the target-facing side are average values, since one cannot exclude minor and unavoidable irregularities in the thickness of the material due to production processes. However, the standard deviation from this average value for the thickness of the target-facing side shall not exceed ±10.0%, preferably not ±5.0% and most preferably not ±2.5% of the average value.

However, in a preferred embodiment of the present invention, the solid particles filling the inner cavity of the inventive RF electrode have a spherical shape, wherein the individual particles have a diameter in the range between 0.05 mm and 20.00 mm, preferably between 0.10 mm and 18.00 mm, preferably between 0.20 mm and 15.00 mm, preferably between 0.50 mm and 12.00 mm, preferably between 1.00 mm and 10.00 mm, preferably between 2.00 mm and 8.00 mm and more preferably between 3.00 mm and 6.00 mm.

### Embodiments - Solid Particles and Liquid Substitute within the Inner Cavity

It has to be emphasized, that in embodiments of the inventive RF electrode, in which solid particles (or caged particles) are used together with a liquid substitute, the solid particles used have at least a 10 times, preferably at least a 20 times, more preferably at least a 30 times, even more preferably at least a 50 times, still more preferably at least a 100 times and most preferably at least a 1000 times larger diameter than the particles forming the liquid substitute. In other words, the solid particles are much bigger than the particles forming the liquid substitute. It shall be noted here, that it is preferred in accordance with the invention, if the diameter of the individual particles forming the liquid substitute is smaller than 50 µm in case the solid particles are larger than 0.5 mm. Thus a maximum size of the particles of the liquid substitute of 50 µm is preferred.

### Liquid Substitute

In accordance with the invention, the **liquid substitute** can be composed of any kind of particles that have similar properties than a real liquid, i.e. there are only low frictional forces between the liquid-like particles forming the liquid substitute which enables them to have similar flow properties than a real liquid. It is furthermore preferred, if the liquid substitute has a good thermal conductivity to serve also for temperature-controlling purposes. The liquid substitute has in general the same functions as the liquid in the embodiments above. The perfusion of the liquid substitute through the inner cavity can be used for temperature-controlling purposes, i.e. cooling or heating the RF electrode and thus the target, such as the skin of the patient, and for supporting the arrangement or move of the solid particles as well as stabilizing the shape assigned to the target-facing side by stabilizing the position and arrangement of the solid particles depending on the volume of liquid substitute present in the inner cavity (see also "sand effect"). Consequently the amount or volume of the liquid substitute present in the inner cavity during the hyperthermia treatment session is normally not constant.

Another embodiment of the present invention is related to a RF electrode with a target-averted side and a mouldable target-facing side and an inner cavity in between, wherein the inner cavity is fillable and emptiable and perfusable with a liquid substitute and the inner cavity contains solid particles arrangeable or movable in the presence of a liquid substitute within the inner cavity and at least a part of the surface of the target-facing side is electrically conductive and is connectable to a RF energy source.

Potential materials the particles forming the liquid substitute partly filling the inner cavity of the inventive RF electrode can be made of comprise: graphite, molybdenum disulfide, polyurethane (PU), polytetrafluoroethylene (PTFE, Teflon^{®}), fluorinated ethylene propylene (FEP), perfluoroalkoxy polymer (PFA), poly(methyl methacrylate) (PMMA), polycarbonate (PC), polyamide (PA), polyvinyl chloride (PVC), polyoxymethylene (POM), polyethylene (PE), polypropylene (PP), polymethylpentene (PMP), polystyrene (PS) and mixtures thereof. However especially preferred materials for the particles forming the liquid substitute are graphite, molybdenum disulfide (MoS₂) and polytetrafluoroethylene (PTFE, Teflon®). Of course, it is also again desirable in accordance with the invention, if the liquid substitute is made of a material that is also biocompatible, i.e. non-toxic to humans or animals, in order to avoid any harm to a patient in the event of an accidental spill.

It is preferred in accordance with the invention, if the individual particles representing the liquid substitute are roughly spherical in shape.

In order to display a flowing behaviour similar to a real liquid it is preferred, if the individual particles forming the liquid substitute are very small. This also enables them to be pumped through the inner cavity of the inventive RF electrode as it is necessary to enable a continuous flow of the liquid substitute during the hyperthermia treatment, i.e. during the treatment time. It is thus preferred in accordance with the invention, if the individual particles forming the liquid substitute should have a diameter preferably below 50 µm, preferably below 30 µm, preferably below 20 µm, more preferably below 10 µm, still more preferably below 5 µm, and still more preferably below 1 µm.

### Solid Particles

Another important aspect of the present embodiments (solid particles and liquid substitute within the inner cavity) according to the invention is again related to the **solid particles** (also referred to as caged particles) filling at least partly the inner cavity of the inventive RF electrode. The solid particles at least partly filling the inner cavity of the inventive RF electrode can be composed of various materials and be present in various shapes and sizes. However, said solid particles (also caged particles) are not able to pass through the at least one inlet or the at least one outlet of the inner cavity of the inventive RF electrode.

Hence in accordance with certain embodiments of the invention, it is preferred to place a filter to both pumping circuit ports (inlet, outlet) to avoid obstruction caused by the solid particles.

In a preferred embodiment of the present invention, the solid particles as a part of the filling of the inner cavity of the inventive RF electrode shall meet the following criteria regarding material properties:
(1) they are not electrically conductive,
(2) they are not magnetic,
(3) they do not stick or adhere together, and do not form aggregates, i.e. they are loose particles,
(4) they keep their shape under the hyperthermia treatment conditions,
(5) they do not change their physical and/or chemical properties (e.g. shape, state of matter) fundamentally in the temperature and pressure range applied according to the invention,

The solid particles have preferably a density below 2.4 g/cm³, preferably a density below 2.2 g/cm³, more preferably a density below 2.0 g/cm³, preferably a density below 1.5 g/cm³, preferably a density below 1.2 g/cm³, preferably a density below 1.1 g/cm³, preferably a density below 1.0 g/cm³, preferably a density below 0.9 g/cm³, preferably a density below 0.8 g/cm³, preferably a density below 0.7 g/cm³, preferably a density below 0.6 g/cm³, preferably a density below 0.5 g/cm³, and preferably a density below 0.4 g/cm³. It is further preferred, if the solid particles have a density between 0.4 g/cm³ - 2.4 g/cm³, more preferably a density between 0.5 g/cm³ - 2.0 g/cm³, more preferably a density between 0.6 g/cm³ - 1.6 g/cm³, yet more preferably a density between 0.7 g/cm³ - 1.4 g/cm³, more preferably a density between 0.8 g/cm³ - 1.2 g/cm³, even more preferably a density between 0.9 g/cm³ - 1.1 g/cm³.

It is especially preferred in accordance with the invention, if the solid particles as a part of the filling of the inventive RF electrode's inner cavity meet all five criteria listed above.

Thus, the present invention is also related to a RF electrode with a target-averted side and a mouldable or deformable target-facing side and an inner cavity in between, wherein the inner cavity is fillable and emptiable and perfusable with a liquid substitute and the inner cavity contains solid particles arrangeable or movable in the presence of a liquid substitute within the inner cavity and at least a part of the surface of the target-facing side is electrically conductive and is connectable to a RF energy source, and the solid particles are loose particles and neither electrically conductive nor magnetic.

Potential materials the solid particles at least partly filling the inner cavity of the inventive RF electrode can be made of comprise: glass, polyurethane (PU), polytetrafluoroethylene (PTFE, Teflon^{®}), fluorinated ethylene propylene (FEP), perfluoroalkoxy polymer (PFA), poly(methyl methacrylate) (PMMA), polycarbonate (PC), polyamide (PA), polyvinyl chloride (PVC), polyoxymethylene (POM), polyethylene (PE), polypropylene (PP), polymethylpentene (PMP), polystyrene (PS) and mixtures thereof. A person skilled in the art is aware of additional materials that fulfil at least most of the above-mentioned criteria and thus can be used in accordance with the invention. Furthermore, it is desired in accordance with the invention, if the material of the solid particles filling at least partly the inner cavity of the inventive RF electrode allows a smooth gliding of said particles against each other (i.e. low friction) and against the liquid substitute in order to promote the adaption to the shape of the target. Of course, it is also desirable in accordance with the invention, if the solid particles are made of a material that is also biocompatible, i.e. non-toxic to humans or animals, in order to avoid any harm to a patient in the event of an accidental spill.

In certain embodiments of the present invention, it is advantageous, if the solid particles filling at least partly the inner cavity of the inventive RF electrode are made from a material that has a lower physical density than the liquid substitute filling the inner cavity of the inventive RF electrode. This helps in reducing the weight of the inventive device and is especially preferred, if the inventive RF electrode is used as an overlay, i.e. is placed on top of the target object.

It is conceivable in accordance with the present invention that the solid particles filling at least partly the inner cavity of the inventive RF electrode can be present in various shapes and sizes.

Possible shapes of the solid particles comprise spheres, rectangles, triangles, and polygons of symmetrical or asymmetrical conformation, but are not limited thereupon. Of course, the solid particles shall have no sharp edges that could pierce the envelope of the inner cavity or that could elicit an unpleasant sensation in a patient. A person skilled in the art is aware of additional shapes that are suitable in accordance with the invention.

However, an important aspect of the present invention regarding the solid particles at least partly filling the inner cavity of the inventive RF electrode is that the solid particles permit a variable but continuous flow of the liquid substitute through the inner cavity of the inventive device during the entire treatment time. As described below in more detail, shape adaption of the inventive RF electrode is facilitated by a purposeful reduction of the liquid substitute's volume inside the inner cavity, while the total volume of the solid particles inside the inner cavity remains constant. Hence, the packaging of the solid particles inside the inner cavity gets denser during shape adaptation. In order to ensure a continuous flow of the liquid substitute it is thus important that interstices between the solid particles persist even after the packaging of said particles gets denser. Hence, it is favourable if the solid particles have a shape that does not tightly interlock, such as for example a spherical shape which is preferred.

The size of the solid particles depends, of course, on the size of the whole RF electrode and also on the volume of the inner cavity. However for all embodiments it can be stated that the solid particles should not have a diameter below 0.05 mm, preferably below 0.10 mm, preferably below 0.20 mm, more preferably below 0.50 mm, still more preferably below 1.00 mm, more preferably below 2.00 mm and preferably below 3.00 mm, more preferably below 4.00 mm and most preferably below 5.00 mm. In addition, the solid particles should not have a diameter exceeding 10.00 mm, preferably exceeding 12.00 mm, preferably exceeding 14.00 mm, preferably exceeding 16.00 mm, preferably exceeding 18.00 mm, preferably exceeding 20.00 mm.

It shall be understood that the values for the sphere diameter are average diameters, since one can not exclude minor irregularities in the shape of the spheres, i.e. one cannot exclude that the spheres are not perfectly round or not perfectly identical to each other. However, the standard deviation from this average value for the sphere diameter shall not exceed ±20.0%, preferably not ±10.0% and most preferably not ±5.0% of the average value.

It shall be mentioned that when used together with a liquid substitute, the solid particles shall have a diameter that is at least 10 times, preferably at least 20 times, more preferably at least 30 times, even more preferably at least 50 times, still more preferably at least 100 times and most preferably at least 1000 times larger than the diameter of the individual particles forming the liquid substitute. It shall be noted here, that it is preferred in accordance with the invention, if the diameter of the individual particles forming the liquid substitute is smaller than 50 µm in case the solid particles are larger than 0.5 mm

The term **"treatment time",** as used herein, refers to the period of time, that follows after the correct placement of the inventive RF electrode and the shape adaptation and in which the target is subjected to the actual RF hyperthermia treatment.

Such a spherical shape of the solid particles at least partly filling the inner cavity of the inventive RF electrode is also favourable with respect to another technical difficulty arising from the packaging of said particles getting denser in the course of shape adaptation: If the solid particles get into closer contact after reduction of the liquid substitute's volume within the inner cavity and the concomitant reduction of the total inner volume of the inner cavity, they might cant depending on shape. Such a cant might lead to unwanted changes of overall shape of the target-facing side of inventive RF electrode.

Thus, in a preferred embodiment of the present invention, the solid particles at least partly filling the inner cavity of the inventive RF electrode have a spherical shape.

In another embodiment of the present invention, holes can be drilled through the solid particles (cf. Fig. 5) to further facilitate the continuous flow of the liquid substitute during the treatment time. However, care shall be taken, that such holes do not jeopardize the stability of said solid particles, so that they do not collapse or break during the operation of the inventive device.

Another aspect of the present invention is related to the size of the solid particles at least partly filling the inner cavity of the inventive RF electrode. There exists an important trade-off that needs to be considered when choosing the size of the solid particles at least partly filling the inner cavity of the inventive RF electrode: The smaller the particles are the smoother is the adaptation to the contours of the target but also the smaller are the interstices between said particles. The latter might impair the flow of the liquid substitute inside the inner cavity of the inventive RF electrode. In turn, the larger the particles are the rougher is the adaptation to the target but the larger are the interstices between the particles. Hence, large particles might facilitate the flow of the liquid substitute inside the inner cavity of the inventive RF electrode, but can impair the adaptation and might in addition elicit an unpleasant sensation of the individual particles in case a living being (human or animal) is the target to be treated. Thus, the choice of particle size shall be made to ensure a sufficient adaptation of the target-facing side of the inventive RF electrode to the shape of the target, while at same time allowing a continuous flow of the liquid substitute inside the inner cavity during the treatment time. Additionally, in case the inventive device is used on a patient (human or animal) an unpleasant sensation due to perception of the particles as such shall be prevented. Especially the latter aspect strongly depends also on the material and thickness of the target-facing side of the RF electrode. In consequence, it is obvious for a person skilled in the art that an optimal particle size depends on the intended application or the precise embodiment of the inventive RF electrode and thus needs to be determined experimentally. However in another embodiment of the present invention, the thickness of the target-facing side is preferably between 0.5 mm - 10.0 mm, still more preferably between 1.0 mm - 8.0 mm, more preferably between 1.5 mm - 7.0 mm, even more preferably between 2.0 mm - 6.0 mm, more preferably between 2.5 mm - 5.0 mm and especially preferred between 3.0 mm - 4.0 mm.

It shall be understood that the values given for the thickness of the target-facing side are average values, since one can not exclude minor and unavoidable irregularities in the thickness of the material due to production processes. However, the standard deviation from this average value for the thickness of the target-facing side shall not exceed ±10.0%, preferably not ±5.0% and most preferably not ±2.5% of the average value.

However, in a preferred embodiment of the present invention, the solid particles at least partly filling the inner cavity of the inventive RF electrode have a spherical shape, wherein the individual particles have a diameter in the range between 0.05 mm and 20.00 mm, preferably between 0.10 mm and 18.00 mm, preferably between 0.20 mm and 15.00 mm, preferably between 0.50 mm and 12.00 mm, preferably between 1.00 mm and 10.00 mm, preferably between 2.00 mm and 8.00 mm and more preferably between 3.00 mm and 6.00 mm.

It is conceivable in another embodiment of the present invention, that in addition to the solid particles (or caged particles) a mixture of a liquid and a liquid substitute as a filling of the inner cavity of the inventive RF electrode. Here, the liquid substitute can act as an additional lubricant allowing a smooth gliding of the solid particles within the inner cavity. Thus, the present invention is also related to a RF electrode with a target-averted side and a mouldable target-facing side and an inner cavity in between, wherein the inner cavity is fillable and emptiable and perfusable with a liquid and a liquid substitute and the inner cavity contains solid particles arrangeable in the presence of a liquid and a liquid substitute within the inner cavity and at least a part of the surface of the target-facing side is electrically conductive and is connectable to a RF energy source, wherein the target-facing side is fittable to any shape of a target by arranging the solid particles within the inner cavity.

It goes without saying that in an embodiment, in which solid particles are used together with a mixture of a liquid and a liquid substitute, that the criteria listed above for the solid particles (see corresponding paragraph in "Embodiments - Solid Particles and Liquid within the Inner Cavity"), the liquid (see corresponding paragraph in "Embodiments - Solid Particles and Liquid within the Inner Cavity") and the liquid substitute (see corresponding paragraph in "Embodiments - Solid Particles and Liquid Substitute within the Inner Cavity") are also valid here. It is especially emphasized that the solid particles used have at least a 10 times, preferably at least a 20 times, more preferably at least a 30 times, even more preferably at least a 50 times, still more preferably at least a 100 times and most preferably at least a 1000 times larger diameter than the particles forming the liquid substitute. In other words, the solid particles are much bigger than the particles forming the liquid substitute.

With respect to the possible constraints, when a liquid is used together with a liquid substitutes it has to be emphasized that the particles forming the liquid substitute shall not stick or adhere together, especially in a wet environment, and do not form aggregates, i.e. they are loose particles. This serves to prevent the liquid substitute from loosing its lubricant effect and also from blocking the inlet or the outlet of the inventive RF electrode. Moreover the particles forming the liquid substitute shall not be swellable in the liquid used. In other words the particles forming the liquid substitute shall be inert in the liquid used.

It has to be emphasized that from here on in the description, the following chapters ("Conductive Layer", "Whole Device", "Mode of Operation", "Possible Applications") of the description refer again to both the embodiments, in which solid particles (or caged particles) are used in combination with a liquid as the filling material of the inner cavity of the inventive RF electrode and in which solid particles (or caged particles) are used in combination with a liquid substitute as the filling material of the inner cavity of the inventive RF electrode and also to the embodiments, in which solid particles (or caged particles) are used in combination with a liquid and a liquid substitute as the filling material of the inner cavity of the inventive RF electrode.

### Conductive Layer

As stated above, the present invention is related to a RF electrode, which target-facing side is mouldable, i.e. which can adapt to the shape of the target's surface. Thus, in order to function as a electrode it is required that at least a part of the surface of the target-facing side of the RF electrode is electrically conductive but of course also flexible and thus able to conform to the contours of the target. A good and close contact of the target-facing surface of the inventive RF electrode to the surface of the target is of vital importance for an efficient and safe energy transfer. Especially in the case that the target is a patient (animal or human) and the inventive RF electrode is used as an overlay, it is additionally of importance that the entire RF electrode, including the target-facing surface, is as light-weight as possible in order to decrease the burden to the patient (animal or human).

Thus, according to an embodiment of the present invention said at least part of the target-facing surface being electrically conductive (conductive layer) is formed from a conductive metal coated flexible material or a metallic network that can be folded or formed freely even into cylinder. Thus, said at least part of the target-facing surface being electrically conductive of the inventive RF electrode can conform to gradual and sharp curvatures.

Thus, in a preferred embodiment of the inventive RF electrode, the electrically conductive part of the target-facing surface of the RF electrode consists of at least one conductive metal electrode material in form of a metallic net or of a coating on a flexible carrier.

Instead of the coated flexible carrier or of the metallic net also a network of metallic fibers can be used.

Hence, a textile is suitable as the flexible carrier, however, any material having flexibility similar to the flexibility of a woven or non-woven textile could also be used in the present invention. Thus, any kind of textile, woven textile, non-woven textile and even non-textile material is suitable as flexible carrier. Such a flexible carrier can also be named as a flexible solid material or flexible solid support. Such carriers, materials or supports are not limited by a specific shape and have the consistency and/or texture of a piece of textile or a piece of fabric or drapery. Consequently all known natural and artificial materials such as polyamide (Nylon^{®}), poly-ε-caprolactone, poly-para-dioxanones, polyanhydrides, polyhydroxymethacrylates, fibrin, polyetherester, polyethylene glycol (PEG), poly(butylene terephthalates), polycarbonates, poly(N-vinyl)-pyrrolidone, polyvinylalcohols, polyesteramides, polyethyleneoxide, polypropyleneoxide, polyurethanes, fibrinogen, starch, collagen, zein, casein, ß-cyclodextrins, polyacrylates, polyacrylamide, polyimides, polyethylene, polypropylene, polytetrafluoroethylene, fluorosilicones, rayon, polysulphones, silicones, polysiloxanes, polyvinyl halogens and copolymers or mixtures of these substances can be used as flexible carrier or material or support according to the present invention.

Preferred are materials, carriers or supports such as these mentioned before which provide a good adhesion for the metallic coating. Also preferred are materials, carriers or supports which are manufactured of or which consist of a plurality of single fibers like a woven textile, wherein one set of the single fibers extends along the complete length of the textile more or less in a substantially parallel manner while the other set of fibers is arranged in a substantially parallel manner diagonal to the first set of fibers. Thus fibers having a length which is similar to the length of the textile comprising said fibers are preferred.

In another embodiment of the present invention the single fibers of the material, carrier or support are coated like a tube that means not only a part of the surface of the fiber is coated but rather the coating is applied all around the fiber.

The conductive metal coating is preferably a multilayer coating. Preferably one of the layers is silver which has a good antibacterial effect and provides for good radiofrequency (RF) conduction. Moreover, silver has an anti-odour effect together with moderate anti-perspiration activity. This makes silver preferred for cosmetic, medical and well-being applications. However other conductive metal coatings can also be used. Preferably the electroless (autocatalytic) deposition of the conductive metal coating is used, which is one of the most frequently used methods for fabricating coatings for corrosion- and wear-resistant purposes.

In a preferred embodiment of the current invention the flexible and conductive part of the target-facing surface of the inventive RF electrode's target-facing side is firmly integrated into the target-facing side of the inventive RF electrode and thus not detachable therefrom. Such a firm integration can be realized for example by directly coating the surface of the target-facing side of the inventive RF electrode with a conductive layer or by gluing, welding or molding said conductive layer into the target-facing side of the RF electrode.

However, in another embodiment of the present invention, the conductive part of the target-facing surface of the RF electrode is present as a detachable layer attached to the target-facing side of the RF electrode. Such a detachable fixation of the conductive layer can be realized for example by means of straps, snap buttons, hook and loop fasteners (Velcro^{®} fasteners), zip fasteners and the like.

The conductive part of the target-facing surface of the inventive RF electrode preferably has no insulation towards the surface of the target (e.g. a patent's skin), so the overall impedance of the system is lower than in a conventional bolus electrode. However, it is conceivable that the conductive part of the target-facing surface of the inventive RF electrode preferably has a very low level of insulation towards the surface of the target (e.g. a patent's skin). Thus, in comparison with a conventional bolus electrode, the use of the same power produces a higher current, which is of course optimal for heating. However, the conductive part of the target-facing surface of the inventive RF electrode shall be insulated towards the liquid containing cavity of the inventive RF electrode.

Another aspect of the present invention is that the conductive layer of the inventive RF electrode is capable to deliver energies of up to 1 kW (totalized over the entire surface of the inventive RF electrode) to a target body. Nevertheless, it is also conceivable that higher energies can be delivered by the conductive layer of the inventive RF electrode to a target body.

However, due to safety reasons the energy applied to the target via the conductive part of the target-facing surface of the inventive RF electrode is usually restricted during the operation of said RF electrode to a value of 0.45 W/cm² per hour.

Hence, another embodiment of the present invention is related to an RF electrode with a target-averted side and a mouldable target-facing side and an inner cavity in between, wherein the inner cavity is fillable and emptiable and perfusable with a liquid and the inner cavity contains solid particles arrangeable or movable in the presence of a liquid within the inner cavity and at least a part of the surface of the target-facing side is electrically conductive and is connectable to an RF energy source, wherein the electrically conductive part of the surface of the target-facing side of the RF electrode consists of at least one conductive metal electrode material in form of a metallic net or a metallic coating.

### Whole device

The present invention also relates to a hyperthermia device comprising the RF electrode according to the present invention and to a capacitor (or condenser) comprising the RF electrode according to the present invention. Most preferably, the inventive RF electrode is the upper electrode in the capacitor and in the hyperthermia device and is lying on the target or on the patient, who is in a horizontal position, while the counter electrode which is the RF electrode below the target or below the patient is a water bed or another inventive RF electrode (cf. Fig. 6).

In other words, the present invention is also related to a RF electrode according to the invention, wherein said RF electrode is integrated into a hyperthermia device.

The term **"hyperthermia",** as used herein, refers to heating up a target or certain parts of a target higher than the environment equilibrium temperature. Thus, the afore-mentioned device comprising the inventive RF electrode is not only suitable to treat humans or animals but also for the hyperthermia treatment of any living and non-living targets and can be used in RF welding, soldering and gluing or in keeping any kind of target material warm such as gases, solids, fluids, liquids, such as dielectric materials.

The RF electrode according to invention can be an electric field coupled electrode (capacitive coupled electrode), a magnetic field coupled electrode (inductive coupled electrode), or a radiative electrode (radiative coupled or antenna array electrode). Preferably the RF electrode of the present invention is an electric field coupled electrode (capacitive coupled electrode).

In order to achieve a capacitive coupling, a capacitor arrangement is necessary, which comprises in the simplest type two electrically conductive elements (e.g. electrodes, RF electrodes) positioned in opposite to each other (usually referred to a as capacitor plates), which are separated by a dielectric (an insulator). The pair of capacitor plates is also sometimes referred to as "electrode" and "counter-electrode".
Thus a preferred embodiment of the present invention is related to the inventive RF electrode, wherein the electrically conductive part of the surface of the target-facing side of the RF electrode is positioned in opposite to at least one counter-electrode or one oppositely charged capacitive electrode.

In other words, a preferred embodiment of the present invention is related to the inventive RF electrode, wherein the RF electrode is part of a capacitor. In that capacitor the inventive RF electrode is the upper electrode which is on top of the target such as a patient while the target is lying in a horizontal position. A counter RF electrode is present in opposite to the upper RF electrode while the target or patient is the dielectric material (dielectricum) in between the two RF electrodes. Thus, the upper RF electrode is arranged in a manner that the electrically conductive part of the surface of the target-facing side of the RF electrode is positioned in opposite to at least one counter-electrode or one oppositely charged capacitive electrode.

As mentioned above, a capacitor arrangement comprises two conductive elements (usually referred to a as capacitor plates, or as electrode and counter-electrode). Thus, it is conceivable in accordance with the present invention that an inventive RF electrode represents only the upper of the two capacitor plates and a different type of electrode represents the lower capacitor plate. However, it is also conceivable that both capacitor plates are represented each by an inventive RF electrode, wherein both inventive RF electrodes do not have to be entirely similar.

Thus, another aspect of the present invention is related to a RF electrode according to the invention, wherein only the upper of the two capacitor plates of a capacitor arrangement is made up by a RF electrode according to the invention or wherein both capacitor plates are made up each by a RF electrode according to the invention.

If the inventive RF electrode is part of a capacitor, the two capacitor plates are arranged or positioned in opposite to each other on the surface of the target (e.g. a patient's skin). The target (e.g. a patient) being positioned in between the at least two capacitor plates becomes part of the capacitor, when the current goes through the target (e.g. a patient) and thus also through the part of the target (e.g. a diseased tissue), which is designated for treatment and which is of course also located in between the two capacitor plates. As a consequence, as already mentioned above, the two capacitor plates (electrode and counter-electrode) should not be insulated or only insulated to a small degree against the surface of the target (e.g. a patient's skin), to which the capacitor plates (electrode and counter electrode) are attached or which is covered by the capacitor plates (electrode or counter electrode).

The direct and close connection of the capacitor plates (electrode and counter electrode) to the surface of the target (e.g. a patient's skin) allows direct current flow through the target (e.g. a patient) which allows to keep the applied voltage lower than in any earlier solutions. In earlier capacitive solutions the electrodes were highly isolated by water boluses (preferable maid from silicone). These highly isolating layers were part of the circuit like serial impedances. The high impedance requests high voltage at definite power, because U²=P*Z, where U is the voltage P is the power and Z is the actual impedance. The current in the same case is low (I²=P/Z, where I is the current). If the isolating layers are eliminated the impedance could be lowered drastically, so that the voltage square lowers and the current square increases proportionally, in order to improve the energy-transfer and suppress the radiation loss. This again is important for focussing the heat at the diseases tissue such as a tumour and to avoid burning of the healthy tissue.

Another important aspect of the present invention is related to a RF electrode according to the invention, wherein the connection to a radiofrequency (RF) energy source is made through a single cable.

Any common radiofrequency (RF) energy source can be used as long as the radiofrequency energy source is able to provide radiofrequency fields in the range of preferably 10 kHz to 50 MHz, and could be safely controlled according to the treatment demands. The range of frequencies supplied can actually range from below the detectable limit (effectively measured as 0 MHz) to 500 MHz, preferably from 10 kHz to 100 MHz, more preferably from 10 kHz to 45 MHz and most preferably 13.56 MHz or any value obtained by multiplication or division by an integer, preferable division by 40. Thus, the following frequencies are most preferred: 13.56 MHz, or 1/100, 1/40, 1/20, 1/10, ½ times, 2 times or 3 times, etc. this value of 13.56 MHz (i.e. 6.78 MHz, 27.12 MHz or 40.68 MHz). The low frequencies are preferred to make definite conduction conditions instead of the radiation of the tissue which easily causes burns in the treated tissue and especially on the skin where the source of radiation is adhered or attached to.

Another embodiment of the present invention is related to the inventive RF electrode, wherein the connection to a radiofrequency (RF) energy source is made via a single cable and wherein the frequency supplied by the radiofrequency (RF) energy source is between 10 kHz and 50 MHz, more preferably between 130 kHz and 42 MHz and most preferably the values 135.6 kHz ± 5%, 339 kHz ± 5%, 678 kHz ± 5%, 1.356 MHz ± 5%, 3.39 MHz ± 5%, 6.78 MHz ± 5%, 13.56 MHz ± 5%, 27.12 MHz ± 5%, and 40.68 MHz ± 5%.

Another embodiment of the present invention is related to the inventive RF electrode, wherein the connection to a radiofrequency (RF) energy source is made through a single cable and wherein the frequency supplied by the radiofrequency (RF) energy source is 13.56 MHz or any value obtained by multiplication or division of 13.56 MHz by an integer, preferable division by 40.

### Mode of Operation - Mechanical & Sand Effect

Another special feature of the present invention is the mode of operation of the inventive RF electrode that facilitates the fitting to the shape (or contour) of a target's surface.

In a conventional bolus electrode (without solid particles), the fluid-filled bolus is usually flexible and can principally adapt to a target up to a certain degree. However, the inner pressure of such a bolus represents a force that counteracts any adaptation in shape. This results in an increased susceptibility of the conventional bolus electrode to slip out of position, a phenomenon which is highly undesirable and poses serious risks to a patient, as already explained above. Thus, force is needed to enable an adaptation of the conventional bolus electrode to the shape of a target body. This force can for example be the weight force of the target (e.g. a patient) lying on top of the bolus electrode. If the bolus electrode is placed on top of the target (i.e. in form of an overlay) the force can be the weight force of the bolus itself and/or an additional force that is applied mechanically by the operating personnel. This force that enables the adaptation of shape against the counteracting inner pressure of the bolus electrode has to be maintained during the entire procedure. This can be a literally heavy burden to a patient, especially if larger areas are to be treated and thus larger and heavier bolus electrodes are needed. As soon as the conventional bolus electrode is detached from the target body it regains its initial shape.

The principle underlying the present invention is quite different from that of the conventional bolus electrode (without solid particles) and optimizes the fitting of the target-facing side of the inventive RF electrode to a target. Maybe the underlying principle can be best illustrated figuratively by a comparison with sand at the beach and is thus also referred to as **"sand effect",** as described briefly earlier. If the water content of the sand is high compared to the total volume of the grains of sand (i.e. an excess of water) the grains of sand are literally swimming in the water. Hence, if one leaves a footprint in such sand, the footprint will rapidly vanish after the foot has been retracted. Something similar will happen if one leaves a footprint in totally dry sand. Instead, if one now considers a wet sand sample with a rather low water content compared to the first example, the grains of sand cannot move as freely as in the first example. If one leaves a footprint in this sand sample, the footprint will persist even after the foot is retracted, of course unless an external force is applied to the footprint.

A more detailed consideration of this will be outlined in the following with respect to the present invention, however the values given are just for exemplification and do not limit the scope of the present invention: In a preferred embodiment of the present invention the ratio between the volume of the liquid (and/or liquid substitute) inside the inner cavity but outside the solid particles (V_{Lo}) and the totalized volume of the solid particles (V_{P}) inside the inner cavity is in the initial flexible state about 4:1. Hence, the solid particles at least filling the inner cavity of the inventive RF electrode have space to move freely and can therefore be easily shifted, i.e. by application of a low force. The transition between a flexible and a stabilized state of the inventive RF electrode is realized by largely reducing the volume of the liquid (and/or liquid substitute) within the inner cavity, while the totalized volume of all solid particles (V_{P}) within the inner cavity remains constant. As a consequence the total inner volume of the inner cavity (Vₜₒₜₐₗ) is reduced. If the inventive RF electrode is placed on top or under a target the reduction of the volume of the liquid (and/or liquid substitute) inside the inner cavity and the concomitant reduction of Vₜₒₜₐₗ strongly promotes the fitting of the target-facing side of the inventive RF electrode to the shape of the target taking advantage of the weight force either of the RF electrode (in case the RF electrode is an overlay) or of the target (in case the RF electrode is placed under the target). At the same time and due to the reduction of Vₜₒₜₐₗ, the density of the solid particles rises inside the inner cavity, as already described above. Hence, said solid particles get more restricted in their movement which confers rigidity to the RF electrode. Thus, in a stabilized state, the inventive RF electrode reversibly retains the inverted shape of the target it has been fitted to (cf. Fig. 1 to 4). By transferring the inventive RF electrode from a flexible into a stabilized state, not only the fitting to a target is promoted but also the assigned shape is stabilized. Nevertheless, a certain degree of flexibility and adaptability remains even in the stabilized state. However, the force needed to change the shape of the target-facing side of the RF electrode in a stabilized state is much higher compared to a flexible state.

Of course, by increasing the volume of the liquid (and/or liquid substitute) inside the inner cavity again, the inventive RF electrode is transferred back into a flexible state and regains its initial shape.

The term "**V**ₜₒₜₐₗ", as used herein, refers to the total inner volume of the inner cavity of the inventive RF electrode taking into account all components that are contained in said inner cavity.

The term "**V_{P}**", as used herein, refers to the totalized volume of **all the solid particles** at least partly filling the inner cavity of the inventive RF electrode and does not refer to the volume of a single solid particle. However, V_{P} does only regard the outer shape of the solid particles and ignores whether the solid particles are full particles (i.e. without drilled holes) or particles with holes drilled through them. That means 10 round solid particles with a diameter of 10 mm that are full have the same V_{P} than 10 round solid particles with a diameter of 10 mm, in which a hole of 1 mm diameter is drilled straight through the middle of each solid particle. Delimited herefrom is "**V_{Ps}**", characterizing only the volume of the solid component of the solid particles and thus taking into account potential drilled holes. That means 10 round solid particles with a diameter of 10 mm that are full have a higher V_{Ps} than 10 round solid particles with a diameter of 10 mm, in which a hole of 1 mm diameter is drilled straight through the middle of each solid particle. It shall be understood, that solid particles that are hollow, but wherein the hollow space is not accessible from the outside of the solid particle, i.e. cannot be filled with a liquid or liquid substitute, are treated as full particles like the solid particle without drilled hole.

The term "**V_{Lo}**", as used herein, refers to the volume of the liquid (and/or liquid substitute) inside the inner cavity of the inventive RF electrode but outside the solid particles also filling said inner cavity. "Outside the solid particles", as used herein, means outside of V_{P}, so again only regards the outer shape of the solid particles irrespective of the absence or presence of holes drilled through the solid particles. Delimited herefrom is **"V_{LP}",** the volume of the liquid (and/or liquid substitute) inside the solid particles, which is zero in case full particles are used as solid particles. In this understanding, the liquid (and/or liquid substitute) filling a hole drilled through a solid particle would be inside that solid particle. Adding up V_{Lo} and V_{LP} results in **"V_{Ltot}",** the total volume of the liquid (and/or liquid substitute) that is contained inside the inner cavity of the inventive RF electrode.

The differentiation between V_{Lo} and V_{LP} and the restriction in the definition of V_{P} ignoring holes drilled through the solid particles is an important aspect, since it allows comparability of preferred volume ratios between V_{Lo} and V_{P} and the resulting total inner volume of the inner cavity (Vₜₒₜₐₗ) irrespective of whether an embodiment of the present invention uses full particles or particles with drilled holes. This is important, because the total volume of Vₜₒₜₐₗ and its change during the course of the fitting process is an important factor for the fitting process according to the invention. If one would choose V_{Ps} and V_{Ltot} as reference volumes to describe the fitting process and one compares the use of full particles with the use of particles with drilled holes, part of V_{Ltot} occupies the holes of the solid particles. As a consequence, using the same bag-like RF electrode one would either end up with a different volume of the inner cavity (Vₜₒₜₐₗ) when using particles with holes instead of full particles, which has an influence on the shape and is thus undesirable, or one would need to apply different volume ratios to achieve the same Vₜₒₜₐₗ.

The latter aspect is illustrated by the following exemplary comparison, wherein an embodiment is described using solid particles and a liquid as the filling of the inner cavity (see also Tab. 1): Given are two identical RF electrodes (case A & B) with a total inner volume of the inner cavity (Vₜₒₜₐₗ) of 500 cm³ in the flexible state. In both cases the solid particles are added in the same amount and with the same diameter, thus a similar V_{P} of 100 cm³. In case A full particles are used (V_{P} = V_{Ps}) and in case B half of the solid particles' volume is represented by a drilled hole (V_{P} = 2 x V_{Ps}). To be able to achieve the same Vₜₒₜₐₗ in both cases, the holes in the solid particles in case B have to be compensated by an increased V_{Ltot}. In both cases of this exemplification Vₜₒₜₐₗ is reduced during the transition from flexible to stabilized state by a factor of 1.67. However, if V_{Ltot} and V_{Ps} are chosen as reference volumes the volume ratio (V_{Ltot} : V_{Ps}) changes during the transition from flexible to stabilized state from 4:1 to 2:1 in case A, but from 9:1 to 5:1 in case B. If, instead, V_{Lo} and V_{P} are used as reference volumes the volume ratio (V_{Lo} : V_{P}) changes are in both cases from 4:1 to 2:1 from flexible to stabilized state.

| **Tab. 1** | | **A) full particles** | | **B) particles with holes** | |
|---|---|---|---|---|---|
| | | *flexible* | *stabilized* | *flexible* | *stabilized* |
| **Liquid** | V_{Ltot} | 400 cm³ | 200 cm³ | 450 cm³ | 250 cm³ |
| | V_{Lo} | 400 cm³ | 200 cm³ | 400 cm³ | 200 cm³ |
| | V_{LP} | 0 cm³ | 0 cm³ | 50 cm³ | 50 cm³ |
| | | | | | |
| **Solid Particles** | V_{P} | 100 cm³ | 100 cm³ | 100 cm³ | 100 cm³ |
| | V_{Ps} | 100 cm³ | 100 cm³ | 50 cm³ | 50 cm³ |
| | Vₜₒₜₐₗ | 500 cm³ | 300 cm³ | 500 cm³ | 300 cm³ |
| | | | | | |
| **Ratio** | V_{Ltot} : V_{Ps} | 4 : 1 | 2 : 1 | 9 : 1 | 5 : 1 |
| | V_{Lo} : V_{P} | 4 : 1 | 2 : 1 | 4 : 1 | 2 : 1 |

The above comparison illustrates that the use of V_{Lo} and V_{P} is best suited to clearly characterize the change of volume ratio during the transition from a flexible to a stabilized state and thus during the inventive fitting process. It shall be mentioned that the definite volume values given in Tab. 1 are just for exemplifying an issue and do not limit the scope of the present application, so the present invention is not limited to these values.

Besides the promoted fitting of the target-facing side of the inventive RF electrode to the shape of the target and the decreased risk of slipping out of position due to the perfect fit, the purposeful reduction of the volume of the liquid (and/or liquid substitute) inside the inner cavity and thus of Vₜₒₜₐₗ also largely reduces the weight of the entire inventive RF electrode. This decreases on the one side the burden to the patient during the treatment and allows on the other side an easier since lighter detachment of the inventive RF electrode from the patient in case of an emergency situation. The decreased weight and the increased safety of the inventive RF electrode also permit the construction of larger RF electrodes that are suitable for the use on entire limbs or even the whole body of a patient.

Another aspect of the present invention is that the RF electrode according to the invention is preferably transferable from a flexible state into a stabilized state thereby stabilizing the assigned shape of the target-facing side.

In other words, another aspect of the present invention is that the RF electrode according to the invention is preferably transferable from a flexible state into a stabilized state by reducing the amount of liquid and/or liquid substitute within the inner cavity.

An alternative way to define the volume changes during the transition from a flexible to a stabilized state of the inventive RF electrode could be to specify that the total inner volume of the inner cavity (Vₜₒₜₐₗ) of the inventive RF electrode is reduced by a factor of 1.2, preferably 1.4, more preferably 1.6, still more preferably 1.8, even more preferably 2.0, more preferably 2.2, more preferably 2.5, and more preferably 2.6 during the transition from the flexible to the stabilized state, while the volume of the solid particles filling the inner cavity remains constant. However, it shall be mentioned here that the total inner volume of the inner cavity (Vₜₒₜₐₗ) shall not be reduced further than to 191% of the totalized volume of all solid particles (V_{P}) within the inner cavity to allow the interstices between the particles to be filled with liquid and/or liquid substitute. Thus, it is obvious for a person skilled in the art that the maximum possible factor of volume reduction depends among others on the volume of the solid particles (V_{P}) and also on the initial volume ratio between the solid particles (V_{P}) and the liquid and/or liquid substitute (V_{Lo}). As an example, in an embodiment of the inventive RF electrode with an initial volume ratio between the solid particles (V_{P}) and the liquid and/or liquid substitute (V_{Lo}) of 1:3 the total inner volume of the inner cavity (Vₜₒₜₐₗ) of the inventive RF electrode can be reduced up to a factor of ca. 2.09 and still fulfil the above requirement (Vₜₒₜₐₗ = 1.91 x V_{P}). In contrast, in an embodiment of the inventive RF electrode with an initial volume ratio between the solid particles (V_{P}) and the liquid and/or liquid substitute (V_{Lo}) of 1:4 the total inner volume of the inner cavity (Vₜₒₜₐₗ) of the inventive RF electrode can be reduced up to a factor of ca. 2.61 and still fulfil the above requirement (Vₜₒₜₐₗ = 1.91 x V_{P}).

In other words, another aspect of the present invention is that the RF electrode according to the invention is transferable from a flexible state into a stabilized state thereby stabilizing the assigned shape of the target-facing side, wherein the total inner volume of the inner cavity (Vₜₒₜₐₗ) of the inventive RF electrode is reduced by, for instance, a factor of 1.2, preferably 1.4, more preferably 1.6, still more preferably 1.8, even more preferably 2.0, more preferably 2.2, more preferably 2.5, and more preferably 2.6 during the transition from flexible to stabilized state, while the totalized volume of all solid particles (V_{P}) inside the inner cavity remains constant and wherein the total inner volume of the inner cavity (Vₜₒₜₐₗ) shall not be reduced further than to 191 % of the totalized volume of all solid particles (V_{P}) within the inner cavity.

However, it is hard to precisely quantify the amount or proportion of the liquid reduction (and/or liquid substitute reduction) during the adaptation process, i.e. when the inventive RF electrode is transferred from a flexible state to a stabilized state, since it does depend on many factors, among them the size of the target to be treated, the initial ratio of solid particles to liquid and/or liquid substitute in flexible state, and the desired degree of adaptation. For example, it might be desirable to adapt the inventive RF electrode in form of an overlay to the torso of a patient. For a patient with a big belly it will be necessary to reduce the volume of the liquid (and/or liquid substitute) inside the inner cavity to a higher extend than for a person with a small belly in order to adapt the electrode to the entire torso, i.e. so that it covers the belly as well as the chest. On the other side, not more liquid (and/or liquid substitute) should be withdrawn than necessary for the desired adaptation, because this might lead to an unwanted crumpling of the target-facing side of the inventive RF electrode and result in a bad fitting with gaps between the target-facing side and the surface of the target. The reduction of the liquid's volume (and/or liquid substitute's volume) inside the inner cavity of the inventive RF electrode has thus to be adjusted to the current treatment situation and especially to the target such as the body shape of a patient which comes into contact with the target-facing side of the electrode. Hence, in a preferred embodiment of the present invention the volume of the liquid and/or the liquid substitute withdrawn during the adaptation process, i.e. when the inventive RF electrode is transferred from a flexible state to a stabilized state, is equal to the volume that the target displaces in order to achieve the desired degree of adaptation (cf. Fig. 1 to 3). However, attention shall be paid, that the volume of liquid and/or liquid substitute withdrawn from the inner cavity during the fitting procedure does preferably not exceed an extent, that not enough liquid (and/or liquid substitute) is left to fill the interstices between the solid particles. This is not only necessary, to ensure a continuous low flow of the liquid and/or the liquid substitute e.g. for temperature-control during the hyperthermia treatment, but also to allow a certain degree of adaptability during the hyperthermia treatment. Thus, the volume of the liquid and/or the liquid substitute (V_{Lo}) within the inner cavity shall preferably only be reduced to a maximal extent in which the volume of the liquid and/or the liquid substitute (V_{Lo}) within the inner cavity is still at least 91% of the totalized volume of all solid particles (V_{P}) within the inner cavity. In other words, the volume of the liquid and/or the liquid substitute (V_{Lo}) within the inner cavity shall only be reduced to a maximal extent in which the total volume of the inner cavity (Vₜₒₜₐₗ) is still at least 191% of the totalized volume of all solid particles (V_{P}) within the inner cavity. Although it is clear from the explanations above, it shall be emphasized here again, that in case not full solid particles but solid particles with drilled holes are used, the volume of the liquid or the liquid substituent necessary to fill these drilled holes (V_{LP}) must not be pumped away and therefore all considerations regarding a reduction of the volume of the liquid and/or liquid substituent within the inner cavity only concern the volume of the liquid and/or liquid substituent within the inner cavity but outside the solid particles (V_{Lo}) and not V_{Ltot}.

Of course, the liquid and/or the liquid substitute shall not be removed from the inner cavity completely.

The term **"flexible"** or **"flexible state",** as used herein, refers to a state of the inventive RF electrode, where the target-facing side of the inventive RF electrode is highly adaptable to a target but regains its initial shape once withdrawn from the target. The reasons for both properties is the high volume of the liquid (and/or liquid substitute) inside the inner cavity of the inventive RF electrode compared to the total volume of all solid particles. That high volume of the liquid enables the solid particles to move more freely inside the inner cavity so that they can be arranged easily.

The term **"stabilized"** or **"stabilized state",** as used herein, refers to a state of the inventive RF electrode, where the shape of the target-facing side of the inventive RF electrode is stabilized due to the reduced volume of the liquid (and/or liquid substitute) inside the inner cavity and the resulting reduced Vₜₒₜₐₗ and the concomitant increase of the solid particle density inside the inner cavity. However, the target-facing side of the inventive RF electrode retains a degree of adaptability even in any "stabilized state", but since the solid particles cannot move as freely as in a "flexible state", the force needed to rearrange them is higher in a "stabilized state" compared to a "flexible state". Thus it is worth to mention that not only one flexible state and one stabilized state exists. There are several flexible states and also several stabilized states. As a not very precise but general rule it could be stated that the higher the volume or the amount of the liquid (and/or the liquid substitute) the more flexible or mouldable the target-facing side. Accordingly the less the amount or volume of the liquid (and/or the liquid substitute) the more stabilized is the shape of the target-facing side. It shall be understood that "flexible" or "flexible state" and "stabilized" or "stabilized state" are no absolute states precisely characterized by e.g. a definite volume ratio between solid particles and liquid and/or liquid substitute, but have to be regarded relative to each other. A reduction in the volume of the liquid and/or liquid substituent (V_{Lo}) filling partly the inner cavity of the inventive RF electrode during the fitting process to a target, while the volume of the solid particles (V_{P}) remains constant, transfers said inventive RF electrode from a (more) flexible state into a (more) stabilized state. The initial volume ratio between solid particles and liquid and/or liquid substitute or the degree of volume reduction during fitting strongly depends on the respective embodiment of the inventive RF electrode and as well on the target to be treated.

Thus, the present invention is also related to a method for the fitting of the inventive RF electrode to a target's surface, comprising the following steps:
a) provision of a RF electrode according to the invention,
b) positioning said RF electrode over the target's surface,
c) making contact of the electrically conductive part of the surface of the target-facing side of the RF electrode with the target's surface by lowering the RF electrode onto the target's surface,
d) transferring the RF electrode from a flexible state into a stabilized state by reducing the volume of the liquid and/or liquid substituent inside the inner cavity of the RF electrode by means of a pumping device, wherein the total volume of all the solid particles within the inner cavity remains constant.

However, it is another aspect of the present invention, that a low pressure flow of the liquid (and/or liquid substitute) through the inner cavity of the inventive RF electrode is maintained during the entire treatment time. This flow of the liquid (and/or liquid substitute) and also the purposeful reduction of the liquid's volume (and/or the liquid substitute's volume) during the fitting procedure can be realized by connecting the at least one inlet and/or the at least one outlet of the inventive RF electrode to at least one adjustable pumping device. A person skilled in the art is aware of methods and devices that are suitable for the use in the present invention. However, in preferred embodiments of the present invention the at least one inlet and/or the at least one outlet of the inventive RF electrode is connected to a pumping circuit that does not only contain a pumping device but also an equalizer tank. This equalizer tank in the pumping circuit serves to take up the amount of liquid and/or liquid substitute withdrawn from the inventive RF electrode during the adaptation to a target.

In another embodiment of the present invention, at least one electronic means is furthermore provided as a part of the inventive RF electrode that generates vibrations and conveys them to the inner cavity of the inventive RF electrode, wherein the power of those vibrations can be adjusted and said means for the generation of vibrations can be switched on and switched off by the operating personnel (e.g. the medical staff).

Thus the present invention is also related to a RF electrode with a target-averted side and a mouldable target-facing side and an inner cavity in between, wherein the inner cavity is fillable and emptiable and perfusable with a liquid and/or a liquid substitute and the inner cavity contains solid particles arrangeable in the presence of a liquid and/or of a liquid substitute within the inner cavity and at least a part of the surface of the target-facing side is electrically conductive and is connectable to a RF energy source, wherein the target-facing side is fittable to any shape of a target by arranging the solid particles within the inner cavity and wherein at least one electronic means is provided for the generation of vibrations in order to support arrangement of the solid particles.

By switching on said electronic means for the generation of vibrations, thereby rattling the inventive RF electrode, during the adaptation process, i.e. when the inventive RF electrode is placed on or under the target, the rearrangement of the solid particles within the inner cavity of the inventive RF electrode and thereby the adaptation process is facilitated. Preferably said electronic means for the generation of vibrations is switched off after adaptation is completed (i.e. after step d) above) and before the hyperthermia treatment begins.

Thus, another embodiment of the present invention is related to a method for the fitting of the inventive RF electrode to a target's surface, as described above by the steps a) to d) comprising the following additional step e):
e) rattling of the inventive RF electrode in order to facilitate the arrangement of the solid particles within the inner cavity of the RF electrode,
wherein step e) can be executed after one or more of the above steps a) to d) or can be executed in parallel to one or more of the above steps a) to d).

A person skilled in the art is aware, that a step comprising the rattling of the inventive RF electrode in order to facilitate the arrangement of the solid particles within the inner cavity of the inventive RF electrode is not necessarily dependent on electronic means for the generation of vibrations, as described above, but can also be conducted by other means, e.g. manually by the operating personnel (e.g. medical personnel).

In a preferred embodiment of the present invention a thermostat is placed somewhere in the supply line delivering the liquid (and/or liquid substitute) to the inlet of the inventive RF electrode. How this is exactly realized is state of the art and known to a person skilled in the art and thus not a part of the present invention. However, in this way, it is possible to control and adjust the temperature of the liquid (and/or liquid substitute) filling the inner cavity of the inventive RF electrode. Hence, it is possible to heat or to cool down the inventive RF electrode as needed. As an example, it might be favourable to slightly warm the inventive RF electrode in order to achieve a temperature that is pleasant to the patient. On the other side, it might be helpful to cool down the inventive device slightly to prevent the patient from sweating during the procedure.

Thus, another embodiment of the present invention is related to an inventive RF electrode, wherein the RF electrode is temperature-controllable by perfusion of the inner cavity with said liquid.

Yet another embodiment of the present invention is related to an inventive RF electrode, wherein the RF electrode is temperature-controllable by perfusion of the inner cavity with said liquid substitute.

Still another embodiment of the present invention is related to an inventive RF electrode, wherein the RF electrode is temperature-controllable by perfusion of the inner cavity with said liquid and said liquid substitute.

Still another embodiment of the present invention is related to an inventive RF electrode, wherein the RF electrode is temperature-controllable by perfusion of the inner cavity with the liquid and/or the liquid substitute.

A person skilled in the art will acknowledge that is difficult to specify parameters of the continuous flow of the liquid (and/or liquid substitute) through the inner cavity of the inventive RF electrode (e.g. flow rate, flow pressure, temperature), since these parameters are strongly dependent on the desired application and their actual conditions.

### Possible Applications

A further aspect of the present invention is the use of a radiofrequency (RF) hyperthermia device including the inventive RF electrode to provide an improved method for treating various medical conditions of a patient (human or animal) including for instance cancer, rheumatoid arthritis, rheumatism, gout, ankylosing spondylitis, lupus, asthma, allergic rhinitis, common cold, fatigue by detoxification of the treated area, muscle spasms and muscle injury.

The cancer can be selected from adenocarcinoma, choroidal melanoma, acute leukemia, acoustic neurinoma, ampullary carcinoma, anal carcinoma, astrocytoma, basal cell carcinoma, pancreatic cancer, desmoid tumour, bladder cancer, bronchial carcinoma, non-small cell lung cancer (NSCLC), breast cancer, Burkitt's lymphoma, corpus cancer, CUP-syndrome (carcinoma of unknown primary), colorectal cancer, small intestine cancer, small intestinal tumours, ovarian cancer, endometrial carcinoma, ependymoma, epithelial cancer types, Ewing's tumours, gastrointestinal tumours, gastric cancer, gallbladder cancer, gall bladder carcinomas, uterine cancer, cervical cancer, glioblastomas, gynecologic tumours, ear, nose and throat tumours, hematologic neoplasias, hairy cell leukemia, urethral cancer, skin cancer, skin testis cancer, brain tumours (gliomas), brain metastases, testicle cancer, hypophysis tumour, carcinoids, Kaposi's sarcoma, laryngeal cancer, germ cell tumour, bone cancer, colorectal carcinoma, head and neck tumours (tumours of the ear, nose and throat area), colon carcinoma, craniopharyngiomas, oral cancer (cancer in the mouth area and on lips), cancer of the central nervous system, liver cancer, liver metastases, leukemia, eyelid tumour, lung cancer, lymph node cancer (Hodgkin's/Non-Hodgkin's), lymphomas, stomach cancer, malignant melanoma, malignant neoplasia, malignant tumours gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, squamous cell carcinoma of the head and neck (SCCHN), prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumours, urologic tumours, urothelial carcinoma, vulva cancer, wart appearance, soft tissue tumours, soft tissue sarcoma, Wilm's tumour, cervical carcinoma and tongue cancer. Particularly suitable for treatment are, for example, astrocytomas, glioblastomas, pancreatic cancer, bronchial cancer, breast cancer, colorectal cancer, ovarian cancer, gastric cancer, laryngeal cancer, malignant melanoma, oesophageal cancer, cervical cancer, liver cancer, bladder cancer, and renal cell cancer.

The RF electrode of the present invention can also be used in combination with other forms of cancer therapy, for example chemotherapy, radiotherapy and surgery.

The inventive RF electrode incorporated into a hyperthermia device can be used in combination with chemotherapy treatment with cytostatic and/or cytotoxic drugs. Examples of some cytostatic and/or cytotoxic drugs are actinomycin D, aminoglutethimide, amsacrin, anastrozol, antagonists of purine and pyrimidine bases, anthracycline, aromatase inhibitors, asparaginase, antiestrogenes, bexaroten, bleomycin, buselerin, busulfan, camptothecin derivates, capecitabin, carboplatin, carmustine, chlorambucil, cisplatin, cladribin, cyclophosphamide, cytarabin, cytosinarabinoside, alkylating cytostatics, dacarbacin, dactinomycin, daunorubicin, docetaxel, doxorubicin (adriamycin), doxorubicin lipo, epirubicin, estramustine, etoposid, exemestan, fludarabin, fluorouracil, folic acid antagonists, formestan, gemcitabin, glucocorticoides, goselerin, hormones and hormone antagonists, hycamtin, hydroxy urea, idarubicin, ifosfamid, imatinib, irinotecan, letrozol, leuprorelin, lomustin, melphalan, mercaptopurine, methotrexate, miltefosin, mitomycine, mitosis inhibitors, mitoxantron, nimustine, oxaliplatin, paclitaxel, pentostatin, procarbacin, tamoxifen, temozolomid, teniposid, testolacton, thiotepa, thioguanine, topoisomerase inhibitors, topotecan, treosulfan, tretinoin, triptorelin, trofosfamide, vinblastine, vincristine, vindesine, vinorelbine, and antibiotics with cytotoxic activities. All present and future cytostatics or other medicaments including gene therapy could be applied as well for a treatment in combination with the inventive RF electrode incorporated into a hyperthermia device.

In case, an embodiment is chosen, in which the inventive RF electrode is in form of a RF electrode for large body parts or even the whole body, the precise site to be treated is selectively chosen automatically from a large volume (e.g. patient's whole body) that could have been treated due to the auto-focussing capabilities of the applied hyperthermia method (described above). The inventive hyperthermia device in form of a whole body hyperthermia device is thus especially useful for the treatment of cancer and of cancer metastases.

Another aspect of the present invention is the use of the inventive RF electrode incorporated into a hyperthermia device to provide an improved method of hyperthermia treatment for upper respiratory tract disease. Upper respiratory tract infections are caused by viruses and bacteria that have an optimum growth and survival temperature lower than the core body temperature. Therefore, these infections can also be treated using hyperthermia therapy. For example, in bacterial infections of the upper part of the respiratory system (e.g. a common cold) the positive effect of heat application is well-known. The mucosa is highly conductive. Similar as in tumour tissue the heat effect is also concentrated (as in asthma treatment). Consequently, the inventive RF electrode incorporated into a hyperthermia device can be used with higher selectivity than other heating techniques. Thus, by relying on an automated focussing due to different impedances of biological tissues the inventive RF electrode incorporated into a hyperthermia device is also useful for the treatment of rhinitis and other upper respiratory tract infections. Examples of viruses that cause upper respiratory tract infections are rhinoviruses, coronaviruses, adenoviruses, myxoviruses, coxsackie viruses, echoviruses, parainfluenza viruses, respiratory syncytial virus and influenza viruses. Examples of bacteria that cause upper respiratory tract infections are *Mycoplasma pneumoniae, Chlamydia pneumoniae, Streptococcus pneumoniae, Corynebacterium diptheriae,* and *Haemophilus influenzae.*

Thus, another aspect of the present invention is the use of the RF electrode according to the invention for the manufacture of a hyperthermia device for the prevention, treatment and after-treatment of cancer, rheumatoid arthritis, rheumatism, gout, ankylosing spondylitis, lupus, asthma, allergic rhinitis, common cold, fatigue by detoxification of the treated area, muscle spasms and/or muscle injury.

Another aspect of the present invention is the use of the RF electrode according to the invention for the manufacture of a hyperthermia device for the prevention, treatment and after-treatment of cancer, rheumatoid arthritis, rheumatism, gout, ankylosing spondylitis, lupus, asthma, allergic rhinitis, common cold, fatigue by detoxification of the treated area, muscle spasms, muscle injury and for cosmetic purposes, for example, cellulite treatment, fat reduction and tissue lifting. Especially preferred is the use of the RF electrode according to the invention for the manufacture of a hyperthermia device for the prevention and treatment of cancer and cancer metastases.

Another aspect of the present invention is the use of the RF electrode according to the invention in the prevention and treatment of cancer, rheumatoid arthritis, rheumatism, gout, ankylosing spondylitis, lupus, asthma, allergic rhinitis, common cold, fatigue by detoxification of the treated area, muscle spasms, muscle injury and for use cosmetic purposes, for example, cellulite treatment, fat reduction and tissue lifting. Especially preferred is the use of the whole body RF electrode according to the invention in the prevention and treatment of cancer and cancer metastases.

Thus, another aspect of the present invention is directed to a method for treating cancer, rheumatoid arthritis, rheumatism, gout, ankylosing spondylitis, lupus, asthma, allergic rhinitis, common cold, fatigue by detoxification of the treated area, muscle spasms, muscle injury, cellulite treatment, or fat reduction of a patient by means of a hyperthermia device, wherein the upper RF electrode has a target-averted side and a mouldable target-facing side and an inner cavity in between, wherein the inner cavity is fillable and emptiable and perfusable with a liquid and/ or liquid substitute and the inner cavity contains solid particles arrangeable or movable in the presence of a liquid and/or liquid substitute within the inner cavity and at least a part of the surface of the target-facing side is electrically conductive and is connectable to a RF energy source, wherein the target-facing side is fittable to any shape of a target by arranging the solid particles within the inner cavity.

When used for treatment of inflammatory conditions the hyperthermia device of the present invention can be used in combination with an anti-inflammatory drug treatment such as a non-steroidal anti-inflammatory drug (NSAID), for example: alcofenac, aceclofenac, sulindac, tolmetin, etodolac, fenopren, thiaprofenic acid, meclofenamic acid, meloxicam, tenoxicam, lornoxicam, nabumetone, acetaminophen, phenacetin, ethenzamide, sulpyrine, mefanamic acid, flufenamic acid, diclofenac sodium, loxoprofen sodium, phenylbutazone, indomethacin, ibuprofen, ketoprofen, naproxen, oxaprozin, flurbiprofen, fenbufen, pranoprofen, floctafenine, piroxicam, epirizole, tiaramide hydrochloride, zaltoprofen, gabexate mesilate, camostat mesilate, ulinastatin, colchicine, probenecid, sulfinpyrazone, benzbromarone, allopurinol, salicylic acid, atropine, scopolamine, levorphanol, ketorolac, tebufelone, tenidap, clofezone, oxyphenbutazone, prexazone, apazone, benzydamine, bucolome, cinchopen, clonixin, ditrazol, epirizole, fenoprofen, floctafenin, glaphenine, indoprofen, niflumic acid and suprofen, or with a steroidal anti-inflammatory drugs, for example, dexamethasone, hexestrol, methimazole, betamethasone, triamcinolone, fluocinonide, prednisolone, methylprednisolone, hydrocortisone, fluorometholone, beclomethasone dipropionate, estriol, clobetasol, diflorasone diacetate, halbetosal propionate, amicinonide, desoximetasone, halcinonide, mometasone furoate, fluticasone propionate, flurandrenolide, clocortalone, predincarbate, aclometasone dipropionate and desonide.

Another aspect of the present invention is the use of the RF electrode according to the invention for cosmetic purposes, for fat reduction for cosmetic purposes and for tissue lifting.

Thus another aspect of the present invention is the use of the inventive RF electrode for cosmetic purposes, fat reduction, cellulite treatment and/or tissue lifting and/or for the prevention, treatment or after-treatment of cancer, rheumatoid arthritis, rheumatism, gout, ankylosing spondylitis, lupus, asthma, allergic rhinitis, common cold, fatigue by detoxification of the treated area, muscle spasms, and/or muscle injury.

Advantages of the present invention are a close and direct contact of the target-facing side of the upper RF electrode to the skin of the patient in order to obtain an optimised capacitive coupled energy transfer by adapting the shape of the target-facing side to the shape of the target thereby avoiding burning the skin of the patient. The present invention allows therewith for the first time the capacitive coupled hyperthermia treatment of large body regions or even the whole body of a patient from the throat to the feet which is especially useful for the treatment of metastases.

### Description of the Figures

Fig. 1: Inventive RF electrode (13) (in form of an overlay) with a rigid target-averted side (5) and a mouldable target-facing side (11) and an inner cavity (3) in between both sides, wherein the inner cavity (3) is partly filled with a liquid and has an inlet (6) and an outlet (7) for said liquid. The inner cavity (3) further contains solid particles (4). Furthermore, an electrically conductive layer is attached to the target-facing side (11) of the RF electrode and forms the electrically conductive part of the surface of the target-facing side (2). The inventive RF electrode is shown in a flexible state before adaptation to the target (1). The solid particles (4) within the inner cavity have space to move and confer flexibility to the structure. The target-facing side (11) is the bottom side of the electrode and the target-averted side (5) is the upper side of the electrode. Moreover a side part (12) is shown connecting the target-facing side (11) to the target-averted side (5) so that the target-facing side (11) together with the target-averted side (5) and the side part (12) form the inner cavity (3).
Fig. 2: Inventive RF electrode as described in Fig. 1 during the fitting process, characterized by a reduction in the liquid volume inside the inner cavity and thus a reduction of the total inner volume of the inner cavity. This volume reduction is accompanied by an increasing density of the solid particles (4) within the inner cavity and an adaptation of the target-facing side (11) of the inventive RF electrode to the surface of the target (1).

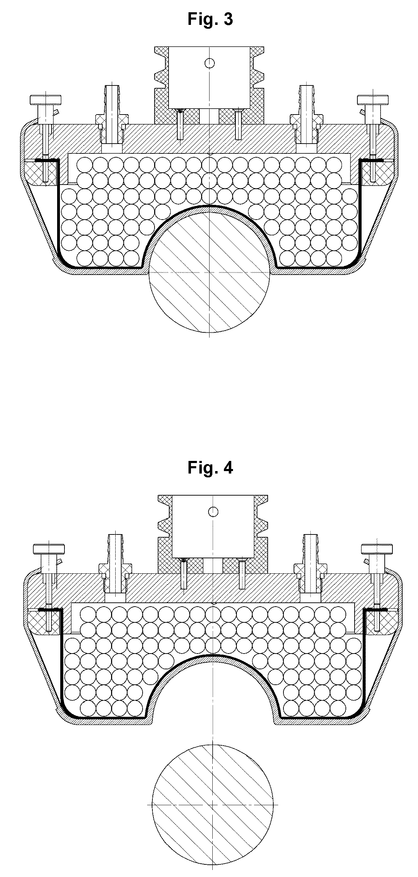

Fig. 3: Inventive RF electrode as described in Fig. 1 after the fitting process, characterized by a further reduction in the liquid volume inside the inner cavity and thus a further reduction of the total inner volume of the inner cavity compared to Fig. 2. This results in a further increased density of the solid particles (4) within the inner cavity and an optimized adaptation of the target-facing side (11) of the inventive RF electrode to the surface of the target (1) going along with stabilization in shape. The target (1) is now engulfed half by the inventive RF electrode.
Fig. 4: Inventive RF electrode as described in Fig. 1 after the fitting process and after withdrawal of the target (1). Due to the reduction in the liquid volume inside the inner cavity, the concomitant reduction of the total inner volume of the inner cavity going along with an increased density of the solid particles (4) within the inner cavity the target-facing side (11) of the inventive RF electrode retains the shape it has been adapted to even though the target (1) is retracted.
**Fig. 5:** Fig. 5 illustrates schematically a single solid particle that can be used in accordance to certain embodiments of the present invention, which is characterized by the fact, that it is not a full particle, but has a hole drilled through its diameter.
**Fig. 6:** Fig. 6 depicts a capacitor arrangement of an inventive RF electrode (13) together with a lower electrode in form of a water bed (14), wherein the target (1) to be treated (here a patient) is placed between the inventive RF electrode (13) and the lower electrode in form of a water bed (14). Both electrodes are connected to an electronic circuit further comprising an RF energy source (9) and additional electronic components (8) that can include e.g. an amplifier, a compensatory circuit, a modulator, a sensor, etc. The inlets show a twofold enlarged area demonstrating the adaptation of the inventive RF electrode in form of an overlay (13) and especially its electrically conductive surface of the target-facing side (2) to the belly region of the patient.

### List of Reference Signs

- 1: target
- 2: electrically conductive part of the surface of the target-facing side (conductive layer)
- 3: inner cavity of the RF electrode
- 4: solid particles
- 5: target-averted side of the RF electrode
- 6: inlet
- 7: outlet
- 8: further electronic components
- 9: RF energy source
- 10: RF energy
- 11: target-facing side of the RF electrode
- 12: side part of the RF electrode
- 13: inventive RF electrode (as upper electrode of a capacitor)
- 14: counter-electrode in form of a water bed

### Examples

### Example 1: Construction type of an inventive RF electrode in form of an overlay

An inventive RF electrode is provided in form of an overlay that has the following technical features, referring to its flexible state: The RF electrode has a roughly cylindrical shape, wherein the target-facing side and the target-averted side represent the bottom face and the top face of the cylinder, respectively. An electrically conductive layer is attached to the target-facing side of the RF electrode and forms the electrically conductive part of the surface of the target-facing side with a diameter of 145 mm and thus a surface area of 165 cm². This electrically conductive layer attached to the target-facing side has a thickness of 0.57 mm and is made of a flexible textile coated with a highly conductive metallic multilayer, wherein the outermost layer of this multilayer (the one that is in direct contact with the target) is silver. The inner cavity, enclosed by the target-facing-side and the target-averted side (together named envelope) has a total inner volume (Vₜₒₜ) of about 1,040 cm³. Said inner cavity has two ports (inlet, outlet) on the target-averted side with an inner diameter of 6 mm allowing the connection of the inner cavity to an external pumping circuit. Those ports have a polypropylene grid (grid distance of 2 mm) on the side of the inner cavity to prevent the solid particles from passing through the ports. Said inner cavity is filled with 6,500 spherical solid particles made of polytetrafluoroethylene (PTFE, Teflon^{®}) and with a diameter of 5 mm. So the totalized volume of all solid particles (V_{P}) inside the inner cavity is 425 cm³ and they are able to occupy the surface area of the electrically conductive layer of the target-facing side in more than 7 layers. The residual volume of the inner cavity not occupied by the solid particles is about 615 cm³ and filled with water containing 25 % ethanol to avoid the growth of germs. Hence the solid particle to liquid ratio is roughly 1 : 1.45 in the non-adapted state. So in this state, the volume of the inner cavity (Vₜₒₜₐₗ) is roughly 2.45 times the totalized volume of all solid particles (V_{P}).

### Example 2: Alternative construction type of an inventive RF electrode in form of an overlay

An inventive RF electrode in form of an overlay according to example 1 is provided, except for the differences that in this embodiment of the invention only 4,000 spherical solid particles made of polytetrafluoroethylene (PTFE, Teflon^{®}) are used. Those solid particles again have a diameter of 5 mm but a hole drilled through their diameter (compare Fig. 5) that occupies 10% of their volume. So in this embodiment the totalized volume of all solid particles (V_{P}) inside the inner cavity is 262 cm³ and given a total inner volume (Vₜₒₜ) of the inner cavity of again 1,040 cm³, the remaining volume within the inner cavity is about 804 cm³, encompassing the liquid volume outside the solid particles (V_{Lo} = 778 cm³) as well as the liquid volume inside the solid particles (V_{LP} = 26 cm³). The holes through the solid particles serve to improve the flow of the liquid through the inner cavity and thereby increase the capability for the exchange of thermal energy.

### Example 3: Operation of an inventive RF electrode in form of an overlay as a part of a hyperthermia device

An inventive RF electrode in form of an overlay according to example 1 is provided for the use in hyperthermia treatment of esophagus cancer in combination with conventional chemotherapy in a human patient that is lying on a water bed representing the lower electrode (or counter electrode) of the entire hyperthermia device.
The RF electrode in form of an overlay (i.e. the upper electrode) is fixed via the rigid target-averted side to a stationary but adjustable lever in a spring-compensated manner. Moreover the inlet and outlet of the RF electrode are connected to a pumping circuit containing a thermostat. The pumping circuit is well exhausted from any air or other gases. By the thermostat in the pumping circuit the temperature of the water/ethanol mixture filling partly the inner cavity of the RF electrode is adjusted to 22 °C. Via the lever the RF electrode is lowered onto the chest region of the patient so that the surface of the target-facing side of the inventive electrode is in contact with the bare skin of the patient. Now, the volume of the water/ethanol mixture within the inner cavity is reduced from ca. 615 cm³ to ca. 440 cm³, i.e. by a factor 1.4, to promote fitting of the RF electrode to the chest of the patient and to transfer the electrode from a flexible state into a stabilized state. The reduction of the liquid within the inner cavity by a factor of 1.4 goes along with a reduction of the volume of the inner cavity (Vₜₒₜₐₗ) by a factor of 1.2. The permanent flow speed of the water/ethanol mixture within the inner cavity is adjusted to 100 cm³/min. Hence, due to the reduction of the volume of the water/ethanol mixture within the inner cavity the surface of the target-facing side of the inventive RF electrode engulfs partly the chest region of the patient, as desired, and adopts the inverted shape of that region. In this way a close and reliable contact between the patient's skin and the electrically conductive surface of the electrode's target-facing side is realized. Both the inventive RF electrode in form of an overlay (upper electrode) and the water bed (lower electrode) are connected to a RF energy supply. The patient is heated by deep hyperthermia with 70 W (13.56 MHz, sinusoidal current, capacitive coupling, RF-current flows through the patient), providing 252 kJ energy (duration is one hour). The hyperthermia treatment is performed in combination with conventional chemotherapy to support the healing of the patient.

## Claims

1. An RF electrode with a target-averted side (5) and a mouldable target-facing side (11) and an inner cavity (3) in between, wherein the inner cavity (3) is fillable and emptiable and perfusable with a liquid and/or a liquid substitute and the inner cavity contains solid particles (4) arrangeable in the presence of a liquid and/or a liquid substitute within the inner cavity (3) and at least a part of the surface (2) of the target-facing side (11) is electrically conductive and is connectable to a RF energy source (9), wherein the target-facing side (11) is fittable to any shape of a target (1) by arranging the solid particles (4) within the inner cavity (3).

2. The RF electrode according to claim 1, wherein the target-averted side (5) is rigid.

3. The RF electrode according to claim 1 or 2, wherein the RF electrode is temperature-controllable by perfusion of the inner cavity (3) with the liquid and/or the liquid substitute.

4. The RF electrode according to any one of the claims 1 - 3, wherein the RF electrode is in form of an overlay placeable on top of the target (1), **characterized in that** the target-facing side (11) is the bottom side of the RF electrode and that the gravitational force of the solid particles (4) inside the inner cavity (3) acts on the inner surface of the target-facing side and is directed to the target (1).

5. The RF electrode according to any one of the claims 1 - 4, wherein the RF electrode is a whole body RF electrode.

6. The RF electrode according to any one of the claims 1 - 5, wherein the liquid is water, ethanol, iso-propanol or mixtures thereof.

7. The RF electrode according to any one of the claims 1 - 6, wherein the solid particles (4) are loose particles which do not adhere or stick to each other, which are neither electrically conductive nor magnetic and if a liquid is present also not soluble in the liquid.

8. The RF electrode according to any one of the claims 1 - 7, wherein the electrically conductive part of the surface of the target-facing side (2) of the RF electrode consists of at least one conductive metal electrode material in form of a metallic net or a metallic coating.

9. The RF electrode according to any one of the claims 1 - 8, wherein said RF electrode further comprises at least one electronic means for the generation of vibrations.

10. The RF electrode according to any one of the claims 1 - 9, wherein the shape of the target-facing side (11) of the RF electrode is transferable from a flexible state into a stabilized state by reducing the amount of liquid and/or liquid substitute within the cavity (3).

11. A capacitor wherein the RF electrode as defined in any one of the claims 1 - 10 is the upper electrode.

12. A hyperthermia device comprising the RF electrode according to any one of the claims 1 -10 or the capacitor of claim 11.

13. The RF electrode according to any one of the claims 1 - 10 or the capacitor of claim 11 or the hyperthermia device of claim 12 for cosmetic purposes, fat reduction, cellulite treatment and/or tissue lifting and/or for the prevention, treatment or aftertreatment of cancer, rheumatoid arthritis, rheumatism, gout, ankylosing spondylitis, lupus, asthma, allergic rhinitis, common cold, fatigue by detoxification of the treated area, muscle spasms, and/or muscle injury.

14. Method for the fitting the RF electrode according to any one of claims 1 - 10 to a target's surface, comprising the following steps:
a) providing a RF electrode according to any one of claims 1 -10,
b) positioning said RF electrode over the target's surface,
c) making contact of the electrically conductive part of the surface of the target-facing side (2) of the RF electrode with the target's surface by lowering the RF electrode onto the target's surface,
d) transferring the RF electrode from a flexible state into a stabilized state by reducing the volume of the liquid and/or liquid substituent inside the inner cavity (3) of the RF electrode by means of a pumping device, wherein the total volume of all the solid particles (4) within the inner cavity (3) remains constant.

15. Method for the fitting the RF electrode according to claim 14 comprising the following additional step e):
e) rattling of the inventive RF electrode in order to facilitate the arrangement of the solid particles (4) within the inner cavity (3) of the RF electrode,
wherein step e) can be executed after one or more of the above steps a) to d) or can be executed in parallel to one or more of the above steps a) to d).

## Patentansprüche

1. Eine RF-Elektrode mit einer dem Ziel abgewandten Seite (5) und einer formbaren dem Ziel zugewandten Seite (11) und einer inneren Kavität (3) dazwischen, wobei die innere Kavität (3) befüllbar und entleerbar und perfusierbar mit einer Flüssigkeit und/oder einem flüssigen Ersatzstoff ist und die innere Kavität feste Partikel (4) enthält, die in Gegenwart von einer Flüssigkeit oder einem flüssigen Ersatzstoff innerhalb der inneren Kavität (3) angeordnet werden können, und mindestens ein Teil der Oberfläche (2) der dem Ziel zugewandten Seite (11) elektrisch leitend und anschließbar an eine RF-Energiequelle (9) ist, wobei die dem Ziel zugewandte Seite (11) anpassbar an jede Form des Ziels (1) durch Anordnen der festen Partikel (4) innerhalb der inneren Kavität (3) ist.

2. Die RF-Elektrode gemäß Anspruch 1, wobei die dem Ziel abgewandte Seite (5) starr ist.

3. Die RF-Elektrode gemäß Anspruch 1 oder 2, wobei die RF-Elektrode temperaturregulierbar mittels Perfusion der inneren Kavität (3) mit der Flüssigkeit und/oder dem flüssigen Ersatzstoff ist.

4. Die RF-Elektrode gemäß einer der Ansprüche 1 - 3 ist, wobei die RF-Elektrode die Form eines Überzuges hat, der auf das Ziel (1) platzierbar ist, charakterisiert dadurch, dass die dem Ziel zugewandte Seite (11) die Unterseite der RF-Elektrode ist und dass die Gravitationskraft der festen Partikel (4) innerhalb der inneren Kavität (3) auf die innere Oberfläche der dem Ziel zugewandten Seite wirken und auf das Ziel (1) gerichtet sind.

5. Die RF-Elektrode gemäß einer der Ansprüche 1 - 4, wobei die RF-Elektrode eine Ganzkörper-RF-Elektrode ist.

6. Die RF-Elektrode gemäß einer der Ansprüche 1 - 5, wobei die Flüssigkeit Wasser, Ethanol, iso-Propanol oder Mischungen davon ist.

7. Die RF-Elektrode gemäß einer der Ansprüche 1 - 6, wobei die festen Partikel (4) lose Partikel sind, die nicht aneinander haften oder kleben, die weder elektrisch leitend noch magnetisch sind und wenn eine Flüssigkeit vorhanden ist, in dieser auch nicht löslich sind.

8. Die RF-Elektrode gemäß einer der Ansprüche 1 - 7, wobei der elektrisch leitende Teil der Oberfläche der dem Ziel zugewandten Seite (2) der RF-Elektrode aus mindestens einem leitenden Metallelektrodenmaterial in Form eines metallischen Netzes oder einer metallischen Beschichtung besteht.

9. Die RF-Elektrode gemäß einer der Ansprüche 1 - 8, wobei besagte RF-Elektrode weiter mindestens ein elektronisches Mittel zur Erzeugung von Schwingungen umfasst.

10. Die RF-Elektrode gemäß einer der Ansprüche 1 - 9, wobei die Form der dem Ziel zugewandten Seite (11) der RF-Elektrode übertragbar ist von einem flexiblen Zustand in einen stabilisierten Zustand durch Reduzierung der Menge an Flüssigkeit und/oder flüssigem Ersatzstoff innerhalb der Kavität (3).

11. Ein Kondensator, wobei die RF-Elektrode definiert nach einer der Ansprüche 1 - 10 die obere Elektrode ist.

12. Eine Hyperthermievorrichtung umfassend die RF-Elektrode gemäß einer der Ansprüche 1 - 10 oder dem Kondensator aus Anspruch 11.

13. Die RF-Elektrode gemäß einer der Ansprüche 1 - 10 oder dem Kondensator aus Anspruch 11 oder der Hyperthermievorrichtung aus Anspruch 12 für kosmetische Zwecke, zur Fettreduktion, Cellulite-Behandlung und/oder Gewebestraffung und/oder zur Prävention, Behandlung oder Nachbehandlung von Krebs, rheumatoider Arthritis, Rheumatismus, Gicht, ankylosierender Spondylitis, Lupus, Asthma, allergische Rhinitis, Erkältung, Müdigkeit durch Entgiftung der behandelten Stelle, Muskelspasmen und/oder Muskelverletzung.

14. Verfahren zur Anpassung der RF-Elektrode gemäß einer der Ansprüche 1 - 10 an die Oberfläche des Ziels, umfassend die folgenden Schritte:
a) Bereitstellen einer RF-Elektrode gemäß einem der Ansprüche 1 - 10,
b) Positionierung besagter RF-Elektrode über der Oberfläche des Ziels,
c) Kontakt herstellen zwischen dem elektrisch leitenden Teil der Oberfläche der dem Ziel zugewandten Seite (2) der RF-Elektrode und der Oberfläche des Ziels durch Absenken der RF-Elektrode auf die Oberfläche des Ziels,
d) Übertragung der RF-Elektrode von einem flexiblen Zustand in einen stabilisierten Zustand durch Reduzierung des Volumens an Flüssigkeit und/oder an flüssigem Ersatzstoff innerhalb der inneren Kavität (3) der RF-Elektrode mittels einer Pumpvorrichtung, wobei das Gesamtvolumen von allen festen Partikeln (4) innerhalb der inneren Kavität (3) konstant bleibt.

15. Verfahren zur Anpassung der RF-Elektrode gemäß Anspruch 14, umfassend den folgenden zusätzlichen Schritt e):
e) Rasseln der erfinderischen RF-Elektrode, um das Anordnen der festen Partikel (4) innerhalb der inneren Kavität (3) der RF-Elektrode zu erleichtern,
wobei Schritt e) nach einem oder mehreren der oben genannten Schritte a) bis d) oder parallel zu einem oder mehreren der oben genannten Schritte a) bis d) ausgeführt werden kann.

## Revendications

1. Une électrode de radiofréquence avec un côté cible-écarté (5) et un côté moulable cible-exposé (11) et une cavité interne (3) entre les deux, où la cavité interne (3) peut être remplie et vidangée et perfusée avec un liquide et/ou un substituant de liquide et la cavité interne contient des particules solides (4) pouvant être agencées en présence d'un liquide et/ou d'un substituant de liquide dans la cavité interne (3) et au moins une partie de la surface (2) du côté cible-exposé (11) est électriquement conductrice et est connectable à une source d'énergie de radiofréquence (9), où le côté cible-exposé (11) est adaptable à n'importe quelle forme de la cible (1) par agencement des particules solides (4) dans la cavité interne (3).

2. L'électrode de radiofréquence selon la revendication 1, où le côté cible-écarté (5) est rigide.

3. L'électrode de radiofréquence selon la revendication 1 ou 2, où l'électrode de radiofréquence est température-contrôlable par perfusion de la cavité interne (3) avec un liquide et/ou un substituant de liquide.

4. L'électrode de radiofréquence selon une quelconque des revendications 1 - 3, où l'électrode de radiofréquence est sous forme d'un recouvrement pouvant être placé au-dessus de la cible (1), **caractérisée en ce que** le côté cible-orienté (11) est le côté inférieur de l'électrode et que la force de gravitation des particules solides (4) dans la cavité interne (3) agit sur la surface interne du côté cible-orienté et est dirigée vers la cible (1).

5. L'électrode de radiofréquence selon une quelconque des revendications 1 - 4, où l'électrode de radiofréquence est une électrode de radiofréquence pour le corps entier.

6. L'électrode de radiofréquence selon une quelconque des revendications 1 - 5, où le liquide est l'eau, l'éthanol, l'isopropanol ou des combinaisons de ceux-ci.

7. L'électrode de radiofréquence selon une quelconque des revendications 1 - 6, où les particules solides (4) sont des particules en vrac, qui n'adhérent pas ou ne collent pas les unes aux autres, qui ne sont pas électriquement conductrices ou magnétiques et si un liquide est présent, elles ne sont pas solubles dans le liquide.

8. L'électrode de radiofréquence selon une quelconque des revendications 1 - 7, où la partie conductrice de la surface du côté cible-écarté (2) de l'électrode de radiofréquence consiste en au moins un matériau d'électrode métallique conductrice sous forme d'un filet métallique ou d'un revêtement métallique.

9. L'électrode de radiofréquence selon une quelconque des revendications 1 - 8, où ladite électrode de radiofréquence comprend de plus au moins un moyen électronique pour la génération des vibrations.

10. L'électrode de radiofréquence selon une quelconque des revendications 1 - 9, où la forme du côté cible-orienté (11) de l'électrode de radiofréquence est transférable d'un état flexible à un état stabilisé par réduction de la quantité de liquide et/ou de substituant de liquide dans la cavité (3).

11. Un condensateur dans lequel l'électrode de radiofréquence telle que définie dans l'une quelconque des revendications 1 - 10 est l'électrode supérieure.

12. Un dispositif d'hyperthermie comprenant l'électrode de radiofréquence selon une quelconque des revendications 1 - 10 ou le condensateur selon la revendication 11.

13. L'électrode de radiofréquence selon une quelconque des revendications 1 - 10 ou le condensateur selon la revendication 11 ou le dispositif d'hyperthermie selon la revendication 12 à des fins cosmétiques, pour la réduction de la graisse, pour le traitement de la cellulite et/ou le soulèvement de tissu et/ou la prévention, le traitement ou post-traitement du cancer, de l'arthrite rhumatoïde, du rhumatisme, de la goutte, de la spondylarthrite ankylosante, du lupus, de l'asthme, de la rhinite allergique, du rhume, de la fatigue par la désintoxication de la zone traitée, des spasmes musculaires et/ou de la blessure musculaire.

14. Procédé pour le raccord de l'électrode de radiofréquence selon l'une quelconque des revendications 1 - 10 à la surface d'une cible, comprenant les étapes suivantes:
a) fournir une électrode de radiofréquence selon l'une quelconque des revendications 1 - 10,
b) positionner ladite électrode de radiofréquence à la surface de la cible,
c) mise en contact de la partie électriquement conductrice de la surface du côté cible-exposé (2) de l'électrode de radiofréquence avec la surface de la cible en abaissant l'électrode de radiofréquence sur la surface de la cible,
d) transférer l'électrode de radiofréquence d'un état flexible à un état stabilisé en réduisant le volume du liquide et/ou du substituant liquide à l'intérieur de la cavité interne (3) de l'électrode de radiofréquence au moyen d'un dispositif de pompage, dans lequel le volume total de toutes les particules solides (4) dans la cavité interne (3) reste constante.

15. Procédé pour le raccord de l'électrode de radiofréquence selon la revendication 14, comprenant de plus l'étape supplémentaire e):
e) cliqueter l'électrode RF selon l'invention afin de faciliter l'agencement des particules solides (4) dans la cavité interne (3) de l'électrode RF,
dans lequel l'étape e) peut être exécutée après une ou plusieurs des étapes ci-dessus a) à d), ou peut être exécutée en parallèle à une ou plusieurs des étapes ci-dessus a) à d).
